# EUROPEAN PATENT APPLICATION

(11) **EP 4 606 910 A1**
(43) Date of publication of application: **27.08.2025**
(21) Application number: 24158907.6
(22) Date of filing: 21.02.2024
(51) Int. Cl.: C12Q 1/6883

(54) **DIAGNOSTIC AND PROGNOSTIC METHODS RELATING TO ULCERATIVE COLITIS**

(71) Applicant: Royal College of Surgeons in Ireland, Dublin 2 (IE); University College Dublin, Belfield, Dublin 4 (IE)
(72) Inventor: DAS, Sudipto, Dublin, Dublin 2 (IE); LAWAL, Ololade, Dublin, Dublin 2 (IE); GENUA, Flavia, Dublin, Dublin 2 (IE); DOHERTY, Glen, Dublin, Dublin 4 (IE)
(74) Representative: Murgitroyd & Company

(57) **Abstract**

The present invention relates to diagnostic and prognostic methods and their use in diagnosing or predicting disease progression in a subject with Ulcerative Colitis (UC). More particularly, the present invention relates to a gene expression signature and the use thereof in determining the likelihood of progression of Ulcerative Colitis in a subject, as well as compositions for the detection thereof. The invention also extends to the use of biomarkers as targets to improve the treatment of Ulcerative Colitis in patients.

## Description

### Field of the invention

The present invention relates to diagnostic and prognostic methods and their use in diagnosing or predicting disease progression in a subject with Ulcerative Colitis (UC). More particularly, the present invention relates to a gene expression signature and the use thereof in determining the likelihood of progression of Ulcerative Colitis in a subject, as well as compositions for the detection thereof. The invention also extends to the use of biomarkers as targets to improve the treatment of Ulcerative Colitis in patients.

### Background to the Invention

Inflammatory bowel disease (IBD) is a term for two conditions (Crohn's disease and Ulcerative Colitis) that are characterized by chronic inflammation of the gastrointestinal (GI) tract. Prolonged inflammation results in damage to the GI tract. Ulcerative Colitis (UC) is a chronic condition that is characterized by profound inflammation of rectal and colonic mucosa. UC has a high global prevalence rate (200-505 per 100,000 individuals)^{[1,2,3]}, which translates into excessive direct and indirect costs, ranging from 12.5-29.1 billion euro annually in Europe alone ^{[2, 4, 5]}. UC is a known relapse-remitting condition and as such there is a pressing demand for new therapeutic modalities for this disease.

People affected by long-standing UC, experience severe inflammation which in turn causes damage to the colonic epithelium^{[1]}. The factors that control the pathogenesis of UC are being elucidated, however full understanding of this process is not complete. Physiologically, UC reverts and remits mucosal inflammation, beginning in the rectum and reaching out to proximal segments of the colon. Endoscopy with biopsies is the only way to determine the diagnosis of ulcerative colitis.

From a treatment point of view, the purpose of current treatment protocols is largely to induce and uphold clinical and endoscopic remission^{[2]}. UC patients are typically treated with various drugs that target to reduce inflammation. Progression of disease in these patients often results in harsh treatment and failure to respond to these treatments ultimately results in surgical removal of inflamed parts of the colon. 5-Aminosalicylates (5-ASA) and/or corticosteroids are the most common first line of treatment for mild-to-moderate UC, and progression of disease results in treatment with steroids to treat UC flares, immunosuppressants and biological drugs. Anti-TNF-α drugs, for example, infliximab, adalimumab, and golimumab, are effective at inducing and maintaining remission in moderate-to-severe-disease. Infliximab can also be used in patients with severe UC and may avoid colectomy^{[3]}. IFX is the most commonly used individually or as part of combination therapy in UC. However, around 40% of patients treated do not respond to infliximab and predictors of response are at present lacking [1,8]

Effective patient management is severely hampered by a lack of unambiguous molecular biomarkers that can predict response to treatment and/or disease progression at an early stage i.e. prior to 5-ASA and/or corticosteroid treatment, and therefore result in personalised treatment of patients and ultimately improve patient quality of life. Potential approaches to identify such biomarkers would require an *indepth* understanding of the genetic, epigenetic, and transcriptomic alterations that drive disease phenotype/pathogenesis and the transcription factors that may be regulating these changes.

There is a significant unmet clinical need to develop accurate biomarker-based methods for patient stratification, which will ultimately enable the identification and development of personalised therapeutic options by predicting disease progression in patients with Ulcerative Colitis at an early stage. The development of a new diagnostic/prognostic assay would allow identification of UC patients who are likely to progress in their disease. This would ultimately enable clinicians to introduce early interventions and save patients from treatment that would ultimately be ineffective and thus improve patient outcome and quality of life. There is also no therapy available for the treatment of UC patients which results in the improved response of patients to existing treatments, such as Infliximab (IFX).

### Summary of Invention

Following extensive experimentation, the present inventors have surprisingly identified methods and the use thereof to predict disease progression in Ulcerative Colitis (UC) patients. More particularly, the inventors have identified a 34-gene expression signature or a subset thereof, which can be used as biomarkers and as a gene signature to enable prediction of disease progression at an early stage for patients with UC. The likelihood of disease progression in a subject with UC may be determined based on the level of expression of one or more of these genes. This will ultimately allow clinicians to make an informed decision in terms of escalating treatment at an early stage of disease onset. The present invention will ultimately spare patients from the side effects of treatment options which are ineffective and thus promotes personalised medicine for the treatment of Irritable Bowel Diseases.

To identify approaches to predict disease progression in UC patients, the inventors carried out detailed transcriptomic analysis for a total of 34 UC patients. To this purpose, the inventors define disease progression as UC patients who required treatment escalation beyond 5 Aminosalicylates (5-ASA) and/or corticosteroid treatment. Disease progression can also be defined as UC patients who require treatment escalation to biologics which would include anti-TNFα targeting agents. These definitions allowed the inventors to stratify the 34 UC patients into: progressors (n=25) and non-progressors (n=9). The inventors detected the expression level of one or more of the genes in the gene expression signature. The inventors' analysis has surprisingly identified a total of 34 differentially expressed genes which are upregulated (34 genes) in UC progressors (n=25) compared to non-progressors (n=9). The expression of these genes is translated to mRNA. The sequences of the mRNA for each gene are outlined as SEQ ID NOs 1-34 in Table 1. Without being bound by theory, the inventors submit that if a subject has the gene expression signature of the present invention, or a subset threof, then they may not benefit from treatment with 5 Aminosalicylates (5-ASA) and/or corticosteroids and may require treatment escalation to biologics which would include anti-TNFα targeting agents.

According to a first aspect of the present invention, there is provided a method for predicting the progression of Ulcerative Colitis in a subject and/or screening for an increased likelihood of progression of Ulcerative Colitis in a subject, the method comprising:
- providing a biological sample which has been obtained from the subject;
- testing the biological sample for the presence or absence of a gene expression signature or a subset thereof;

wherein the gene expression signature, or a subset thereof, comprises or consists of one or more of the genes set forth in Table 1;
wherein the presence of the gene expression signature, or a subset thereof, indicates an increased likelihood of disease progression.

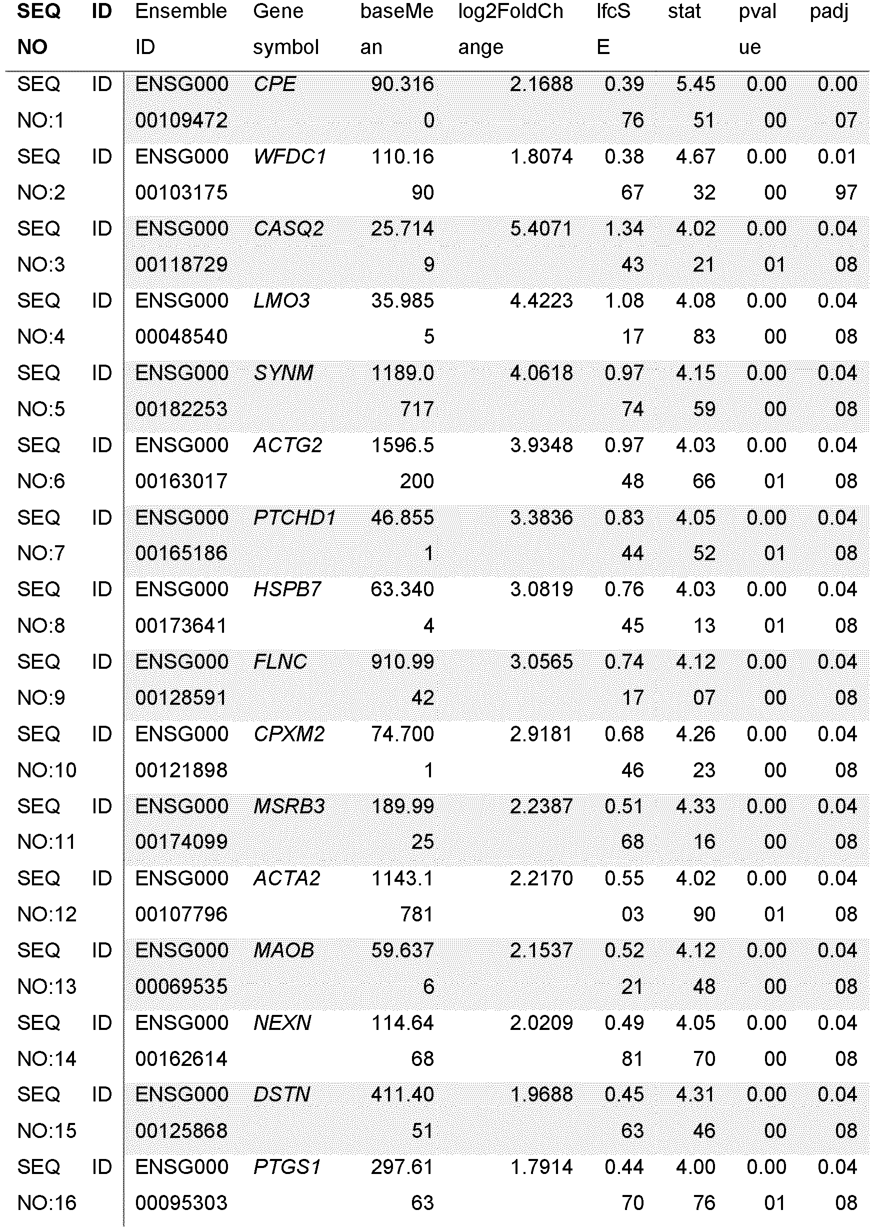

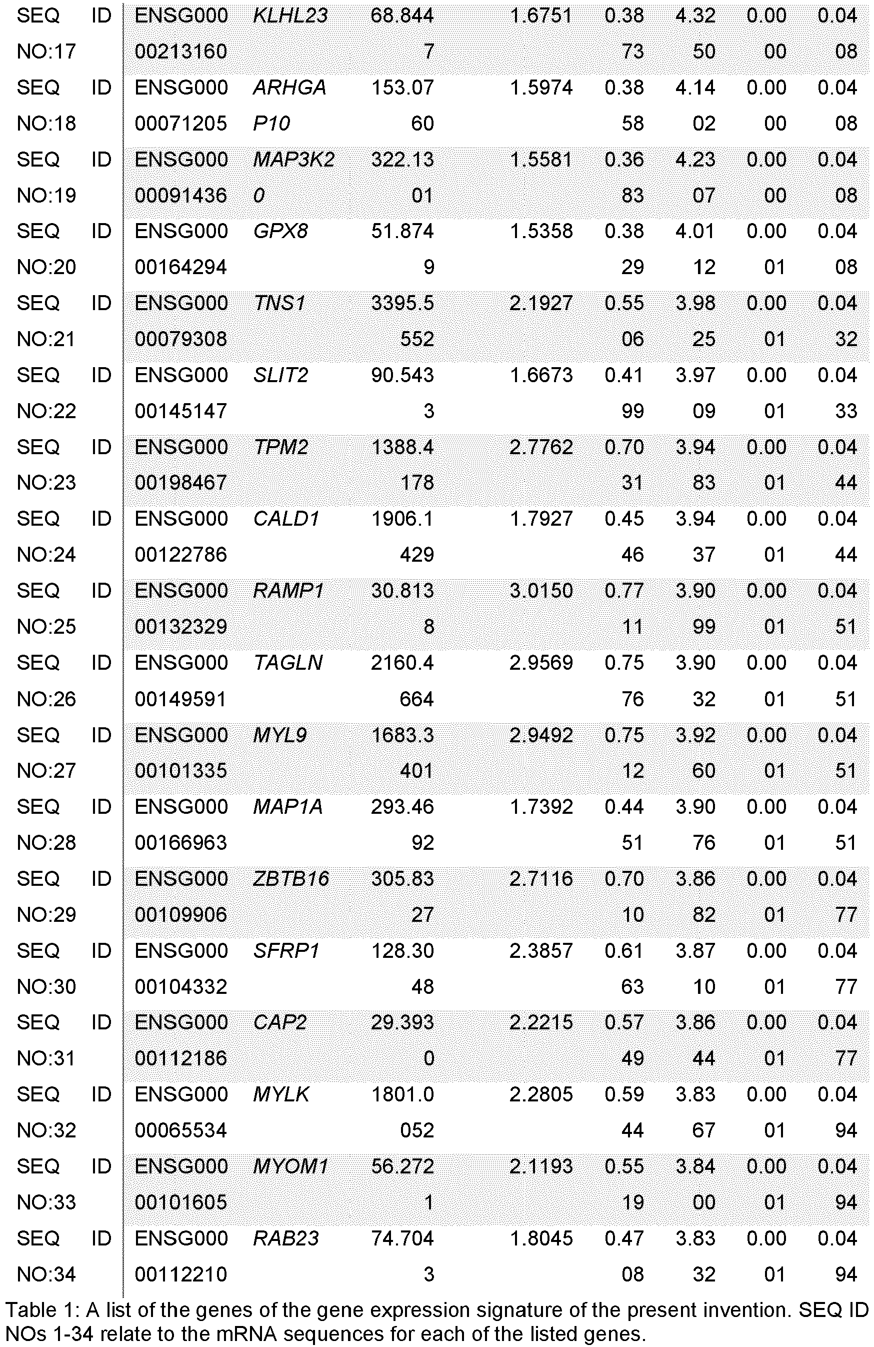

In embodiments, the gene expression signature or a subset thereof comprises or consists of one or more genes selected from the group comprising or consisting of carboxypeptidase E (*CPE*), WAP four-disulfide core domain 1 (*WFDC1*)*,* Calsequestrin 2 (*CASQ*2)*,* LIM domain only 3 (*LMO3*)*,* Synemin (*SYNM*)*,* Actin Gamma 2, smooth muscle (*ACTG2*), Patched domain containing 1 (*PTCHD1*)*,* Heat Shock protein family B (small) member 7 (*HSPB7*)*,* Filamin C (*FLNC*)*,* Carboxypeptidase X, M14 family member 2 (*CPXM*2)*,* Methionine sulfoxide reductase B3 (*MSRB3*)*,* Actin alpha 2, smooth muscle (*ACTA2*), Monoamine oxidase B (*MAOB*)*,* Nexilin F-actin binding protein (*NEXN*)*,* Destrin, actin depolymerizing factor (*DSTN*)*,* Prostaglandin-endoperoxide synthase 1 (*PTGS1*)*,* Kelch like family member 23 (*KLHL23*)*,* Rho GTPase activating protein 10 (*ARHGAP10*)*,* Mitogen-activated protein kinase kinase kinase 20 (*MAP3K20*)*,* Glutathione peroxidase 8 (GPX8), Tensin 1 (*TNS1*)*,* Slit guidance ligand 2 (*SLIT2*)*,* Tropomyosin 2 (*TPM2*)*,* Caldesmon 1 (*CALD1*)*,* Receptor activity modifying protein 1 (*RAMP1*)*,* Transgelin (*TAGLN*)*,* Myosin Light chain 9 (*MYL9*)*,* Microtubule associated protein 1A (*MAP1A*)*,* Zinc finger and BTB domain containing 16 (*ZBTB16*)*,* Secreted frizzled related protein 1 (*SFRP1*)*,* Cyclase associated actin cytoskeleton regulatory protein 2 (*CAP2*), Myosin light chain kinase (*MYLK*)*,* Myomesin 1 (*MYOM1*) and RAS oncogene family (*RAB23*)*.*

In embodiments, the gene expression signature comprises or consists of the mRNA sequences of the one or more genes and the mRNA sequences comprise or consist of the nucleic acid sequences of SEQ ID NO:1 to SEQ ID NO:34 or functionally active fragments or variants thereof; or any combination thereof.

In embodiments, the gene expression signature comprises or consists of one or more genes selected from the group comprising or consisting of Carboxypeptidase E (*CPE*)*,* Glutathione Peroxidase 8 (*GPX8*) and Zinc Finger and BTB Domain Containing 16 (*ZBTB16*)*.* In embodiments, the gene expression signature comprises or consists of the mRNA sequences of the one or more genes and the mRNA sequences comprise or consist of the nucleic acid sequences of SEQ ID NO:1 or a functionally active fragment or variant thereof, SEQ ID NO:20 or a functionally active fragment or variant thereof or SEQ ID NO:29 or a functionally active fragment or variant thereof; or any combination of SEQ ID NO:1, SEQ ID NO:20 or SEQ ID NO:29 or a functionally active fragments or variants thereof.

In embodiments, the gene expression signature comprises or consists of one or more genes selected from the group comprising or consisting of Mitogen-activated protein kinase kinase kinase 20 (*MAP3K20*)*,* Rho GTPase activating protein 1 (*ARHGAP10*) and Prostaglandin-endoperoxide synthase 1 (*PTGS1*)*.* In embodiments, the gene expression signature comprises or consists of the mRNA sequences of the one or more genes and the mRNA sequences comprise or consist of the nucleic acid sequences of SEQ ID NO: 19 or a functionally active fragment or variant thereof, SEQ ID NO: 18 or a functionally active fragment or variant thereof or SEQ ID NO: 16 or a functionally active fragment or variant thereof; or any combination of SEQ ID NO:19, SEQ ID NO:18 or SEQ ID NO:16 or a functionally active fragments or variants thereof.

In embodiments, the gene expression signature, or the subset thereof, of the present invention comprises or consists of at least 98%, at least 95%, at least 90%, at least 80%, at least 70%, at least 60%, at least 50%, at least 40%, at least 30%, at least 20%, at least 10%, at least 5% or at least 1% of the genes set forth in Table 1.

In embodiments, the one or more genes are upregulated.

In embodiments, the progression of Ulcerative Colitis is defined as subjects who require treatment that extends beyond 5 Aminosalicylates (5-ASA) and/or corticosteroid treatment. In embodiments, the progression of Ulcerative Colitis is defined as subjects who require treatment escalation to biologics which would include anti-TNFα targeting agents. In embodiments, the progression of Ulcerative Colitis is defined as subjects who require treatment that extends beyond 5 Aminosalicylates (5-ASA) and/or corticosteroid treatment and who require treatment escalation to biologics which would include anti-TNFα targeting agents. In embodiments, the progression of Ulcerative Colitis in a subject is from mild to moderate to severe.

In embodiments, the presence of the gene expression signature or a subset thereof is determined by a method of gene expression profiling.

According to a further aspect of the present invention, there is provided a method of using a gene expression signature or a subset thereof in a subject as a biomarker to evaluate the likelihood that a therapy for Ulcerative Colitis would be effective in the subject, the method comprising:
- providing a biological sample which has been obtained from the subject; and
- testing the biological sample for the presence or absence of a gene expression signature or a subset thereof;

wherein the gene expression signature or a subset thereof comprises or consists of one or more of the genes listed in Table 1;
wherein the presence of the gene expression signature or a subset thereof indicates that the therapy should be avoided.

In embodiments, the therapy is 5-aminosalicylate acid (5-ASA) or corticosteroid treatment. In embodiments, the therapy is 5-aminosalicylate acid (5-ASA) and corticosteroid treatment.

In embodiments, the gene expression signature or a subset thereof comprises or consists of one or more genes selected from the group comprising or consisting of carboxypeptidase E (*CPE*), WAP four-disulfide core domain 1 (*WFDC1*)*,* Calsequestrin 2 (*CASQ2*)*,* LIM domain only 3 (*LMO3*)*,* Synemin (*SYNM*)*,* Actin Gamma 2, smooth muscle (*ACTG2*), Patched domain containing 1 (*PTCHD1*)*,* Heat Shock protein family B (small) member 7 (*HSPB7*)*,* Filamin C (*FLNC*)*,* Carboxypeptidase X, M14 family member 2 (*CPXM2*)*,* Methionine sulfoxide reductase B3 (*MSRB3*)*,* Actin alpha 2, smooth muscle (*ACTA2*), Monoamine oxidase B (*MAOB*)*,* Nexilin F-actin binding protein (*NEXN*)*,* Destrin, actin depolymerizing factor (*DSTN*)*,* Prostaglandin-endoperoxide synthase 1 (*PTGS1*)*,* Kelch like family member 23 (*KLHL23*)*,* Rho GTPase activating protein 10 (*ARHGAP10*)*,* Mitogen-activated protein kinase kinase kinase 20 (*MAP3K20*)*,* Glutathione peroxidase 8 (GPX8), Tensin 1 (*TNS1*)*,* Slit guidance ligand 2 (*SLIT2*)*,* Tropomyosin 2 (*TPM2*)*,* Caldesmon 1 (*CALD1*)*,* Receptor activity modifying protein 1 (*RAMP1*)*,* Transgelin (*TAGLN*)*,* Myosin Light chain 9 (*MYL9*)*,* Microtubule associated protein 1A (*MAP1A*)*,* Zinc finger and BTB domain containing 16 (*ZBTB16*)*,* Secreted frizzled related protein 1 (*SFRP1*)*,* Cyclase associated actin cytoskeleton regulatory protein 2 (*CAP2*), Myosin light chain kinase (*MYLK*)*,* Myomesin 1 (*MYOM1*) and RAS oncogene family (*RAB23*)*.*

In embodiments, the gene expression signature comprises or consists of the mRNA sequences of the one or more genes and the mRNA sequences comprise or consist of the nucleic acid sequences of SEQ ID NO:1 to SEQ ID NO:34 or functionally active fragments or variants thereof; or any combination thereof.

In embodiments, the gene expression signature comprises or consists of one or more genes selected from the group comprising or consisting of Carboxypeptidase E (*CPE*), Glutathione Peroxidase 8 (*GPX8*) and Zinc Finger and BTB Domain Containing 16 (*ZBTB16*)*.* In embodiments, the gene expression signature comprises or consists of the mRNA sequences of the one or more genes and the mRNA sequences comprise or consist of the nucleic acid sequences of SEQ ID NO:1 or a functionally active fragment or variant thereof, SEQ ID NO:20 or a functionally active fragment or variant thereof or SEQ ID NO:29 or a functionally active fragment or variant thereof; or any combination of SEQ ID NO:1, SEQ ID NO:20 or SEQ ID NO:29 or a functionally active fragments or variants thereof.

In embodiments, the gene expression signature comprises or consists of one or more genes selected from the group comprising or consisting of Mitogen-activated protein kinase kinase kinase 20 (*MAP3K20*)*,* Rho GTPase activating protein 1 (*ARHGAP10*) and Prostaglandin-endoperoxide synthase 1 (*PTGS1*)*.* In embodiments, the gene expression signature comprises or consists of the mRNA sequences of the one or more genes and the mRNA sequences comprise or consist of the nucleic acid sequences of SEQ ID NO:19 or a functionally active fragment or variant thereof, SEQ ID NO:18 or a functionally active fragment or variant thereof or SEQ ID NO:16 or a functionally active fragment or variant thereof; or any combination of SEQ ID NO:19, SEQ ID NO:18 or SEQ ID NO:16 or a functionally active fragments or variants thereof.

In embodiments, the gene expression signature, or the subset thereof, of the present invention comprises or consists of at least 98%, at least 95%, at least 90%, at least 80%, at least 70%, at least 60%, at least 50%, at least 40%, at least 30%, at least 20%, at least 10%, at least 5% or at least 1% of the genes set forth in Table 1.

In embodiments, the one or more genes are upregulated.

In embodiments, the progression of Ulcerative Colitis is defined as subjects who require treatment that extends beyond 5 Aminosalicylates (5-ASA) and/or corticosteroid treatment. In embodiments, the progression of Ulcerative Colitis is defined as subjects who require treatment escalation to biologics which would include anti-TNFα targeting agents. In embodiments, the progression of Ulcerative Colitis is defined as subjects who require treatment that extends beyond 5 Aminosalicylates (5-ASA) and/or corticosteroid treatment and who require treatment escalation to biologics which would include anti-TNFα targeting agents. In embodiments, the progression of Ulcerative Colitis in a subject is from mild to moderate to severe.

According to a further aspect of the present invention, there is provided a method of predicting the responsiveness of a patient suffering from UC to a particular treatment or a method for determining if the treatment of a patient with UC should be escalated, the method comprising:
- providing a biological sample which has been obtained from the subject; and
- testing the biological sample for the presence or absence of a gene expression signature or a subset thereof;

wherein the gene expression signature or a subset thereof comprises or consists of one or more of the genes listed in Table 1;
wherein the presence of the gene expression signature or a subset thereof indicates that the therapy should be avoided.

In embodiments, the therapy is 5-aminosalicylate acid (5-ASA) or corticosteroid treatment. In embodiments, the therapy is 5-aminosalicylate acid (5-ASA) and corticosteroid treatment.

In embodiments, the gene expression signature or a subset thereof comprises or consists of one or more genes selected from the group comprising or consisting of carboxypeptidase E (*CPE*), WAP four-disulfide core domain 1 (*WFDC1*)*,* Calsequestrin 2 (*CASQ2*)*,* LIM domain only 3 (*LMO3*)*,* Synemin (*SYNM*)*,* Actin Gamma 2, smooth muscle (*ACTG2*), Patched domain containing 1 (*PTCHD1*)*,* Heat Shock protein family B (small) member 7 (*HSPB7*)*,* Filamin C (*FLNC*)*,* Carboxypeptidase X, M14 family member 2 (*CPXM2*)*,* Methionine sulfoxide reductase B3 (*MSRB3*)*,* Actin alpha 2, smooth muscle (*ACTA2*), Monoamine oxidase B (*MAOB*)*,* Nexilin F-actin binding protein (*NEXN*)*,* Destrin, actin depolymerizing factor (*DSTN*)*,* Prostaglandin-endoperoxide synthase 1 (*PTGS1*)*,* Kelch like family member 23 (*KLHL23*)*,* Rho GTPase activating protein 10 (*ARHGAP10*)*,* Mitogen-activated protein kinase kinase kinase 20 (*MAP3K20*)*,* Glutathione peroxidase 8 (GPX8), Tensin 1 (*TNS1*)*,* Slit guidance ligand 2 (*SLIT2*)*,* Tropomyosin 2 (*TPM2*)*,* Caldesmon 1 (*CALD1*)*,* Receptor activity modifying protein 1 (*RAMP1*)*,* Transgelin (*TAGLN*)*,* Myosin Light chain 9 (*MYL9*)*,* Microtubule associated protein 1A (*MAP1A*)*,* Zinc finger and BTB domain containing 16 (*ZBTB16*)*,* Secreted frizzled related protein 1 (*SFRP1*)*,* Cyclase associated actin cytoskeleton regulatory protein 2 (*CAP2*), Myosin light chain kinase (*MYLK*)*,* Myomesin 1 (*MYOM1*) and RAS oncogene family (*RAB23*)*.*

In embodiments, the gene expression signature comprises or consists of the mRNA sequences of the one or more genes and the mRNA sequences comprise or consist of the nucleic acid sequences of SEQ ID NO:1 to SEQ ID NO:34 or functionally active fragments or variants thereof; or any combination thereof.

In embodiments, the gene expression signature comprises or consists of one or more genes selected from the group comprising or consisting of Carboxypeptidase E (*CPE*), Glutathione Peroxidase 8 (*GPX8*) and Zinc Finger and BTB Domain Containing 16 (*ZBTB16*)*.* In embodiments, the gene expression signature comprises or consists of the mRNA sequences of the one or more genes and the mRNA sequences comprise or consist of the nucleic acid sequences of SEQ ID NO:1 or a functionally active fragment or variant thereof, SEQ ID NO:20 or a functionally active fragment or variant thereof or SEQ ID NO:29 or a functionally active fragment or variant thereof; or any combination of SEQ ID NO:1, SEQ ID NO:20 or SEQ ID NO:29 or a functionally active fragments or variants thereof.

In embodiments, the gene expression signature comprises or consists of one or more genes selected from the group comprising or consisting of Mitogen-activated protein kinase kinase kinase 20 (*MAP3K20*)*,* Rho GTPase activating protein 1 (*ARHGAP10*) and Prostaglandin-endoperoxide synthase 1 (*PTGS1*)*.* In embodiments, the gene expression signature comprises or consists of the mRNA sequences of the one or more genes and the mRNA sequences comprise or consist of the nucleic acid sequences of SEQ ID NO:19 or a functionally active fragment or variant thereof, SEQ ID NO:18 or a functionally active fragment or variant thereof or SEQ ID NO:16 or a functionally active fragment or variant thereof; or any combination of SEQ ID NO:19, SEQ ID NO:18 or SEQ ID NO:16 or a functionally active fragments or variants thereof.

In embodiments, the gene expression signature, or the subset thereof, of the present invention comprises or consists of at least 98%, at least 95%, at least 90%, at least 80%, at least 70%, at least 60%, at least 50%, at least 40%, at least 30%, at least 20%, at least 10%, at least 5% or at least 1% of the genes set forth in Table 1.

In embodiments, the one or more genes are upregulated.

In embodiments, the progression of Ulcerative Colitis is defined as subjects who require treatment that extends beyond 5 Aminosalicylates (5-ASA) and/or corticosteroid treatment. In embodiments, the progression of Ulcerative Colitis is defined as subjects who require treatment escalation to biologics which would include anti-TNFα targeting agents. In embodiments, the progression of Ulcerative Colitis is defined as subjects who require treatment that extends beyond 5 Aminosalicylates (5-ASA) and/or corticosteroid treatment and who require treatment escalation to biologics which would include anti-TNFα targeting agents. In embodiments, the progression of Ulcerative Colitis in a subject is from mild to moderate to severe.

According to a further aspect of the present invention, there is provided a method of treatment and/or prophylaxis of Ulcerative Colitis in a subject in need thereof, the method comprising the steps of:
(a) determining the presence or absence of a gene expression signature or a subset thereof in a biological sample obtained from said subject;
   wherein the gene expression signature or a subset thereof comprises or consists of one or more of the genes listed in Table 1;
   wherein the presence of the gene expression signature or a subset thereof indicates an increased likelihood of disease progression in the subject from which the test sample was obtained; and
(b) administering to said subject an effective amount of an Ulcerative Colitis therapeutic agent.

In embodiments, the gene expression signature or a subset thereof comprises or consists of one or more genes selected from the group comprising or consisting of carboxypeptidase E (*CPE*), WAP four-disulfide core domain 1 (*WFDC1*)*,* Calsequestrin 2 (*CASQ2*)*,* LIM domain only 3 (*LMO3*)*,* Synemin (*SYNM*)*,* Actin Gamma 2, smooth muscle (*ACTG2*), Patched domain containing 1 (*PTCHD1*)*,* Heat Shock protein family B (small) member 7 (*HSPB7*)*,* Filamin C (*FLNC*)*,* Carboxypeptidase X, M14 family member 2 (*CPXM2*)*,* Methionine sulfoxide reductase B3 (*MSRB3*)*,* Actin alpha 2, smooth muscle (*ACTA2*), Monoamine oxidase B (*MAOB*)*,* Nexilin F-actin binding protein (*NEXN*)*,* Destrin, actin depolymerizing factor (*DSTN*)*,* Prostaglandin-endoperoxide synthase 1 (*PTGS1*)*,* Kelch like family member 23 (*KLHL23*)*,* Rho GTPase activating protein 10 (*ARHGAP10*)*,* Mitogen-activated protein kinase kinase kinase 20 (*MAP3K20*)*,* Glutathione peroxidase 8 (GPX8), Tensin 1 (*TNS1*)*,* Slit guidance ligand 2 (*SLIT2*)*,* Tropomyosin 2 (*TPM2*)*,* Caldesmon 1 (*CALD1*)*,* Receptor activity modifying protein 1 (*RAMP1*)*,* Transgelin (*TAGLN*)*,* Myosin Light chain 9 (*MYL9*)*,* Microtubule associated protein 1A (*MAP1A*)*,* Zinc finger and BTB domain containing 16 (*ZBTB16*)*,* Secreted frizzled related protein 1 (*SFRP1*)*,* Cyclase associated actin cytoskeleton regulatory protein 2 (*CAP2*), Myosin light chain kinase (*MYLK*)*,* Myomesin 1 (*MYOM1*) and RAS oncogene family (*RAB23*)*.*

In embodiments, the gene expression signature comprises or consists of the mRNA sequences of the one or more genes and the mRNA sequences comprise or consist of the nucleic acid sequences of SEQ ID NO:1 to SEQ ID NO:34 or functionally active fragments or variants thereof; or any combination thereof.

In embodiments, the gene expression signature comprises or consists of one or more genes selected from the group comprising or consisting of Carboxypeptidase E (*CPE*)*,* Glutathione Peroxidase 8 (*GPX8*) and Zinc Finger and BTB Domain Containing 16 (*ZBTB16*)*.* In embodiments, the gene expression signature comprises or consists of the mRNA sequences of the one or more genes and the mRNA sequences comprise or consist of the nucleic acid sequences of SEQ ID NO:1 or a functionally active fragment or variant thereof, SEQ ID NO:20 or a functionally active fragment or variant thereof or SEQ ID NO:29 or a functionally active fragment or variant thereof; or any combination of SEQ ID NO:1, SEQ ID NO:20 or SEQ ID NO:29 or a functionally active fragments or variants thereof.

In embodiments, the gene expression signature comprises or consists of one or more genes selected from the group comprising or consisting of Mitogen-activated protein kinase kinase kinase 20 (*MAP3K20*)*,* Rho GTPase activating protein 1 (*ARHGAP10*) and Prostaglandin-endoperoxide synthase 1 (*PTGS1*)*.* In embodiments, the gene expression signature comprises or consists of the mRNA sequences of the one or more genes and the mRNA sequences comprise or consist of the nucleic acid sequences of SEQ ID NO:19 or a functionally active fragment or variant thereof, SEQ ID NO:18 or a functionally active fragment or variant thereof or SEQ ID NO:16 or a functionally active fragment or variant thereof; or any combination of SEQ ID NO:19, SEQ ID NO:18 or SEQ ID NO:16 or a functionally active fragments or variants thereof.

In embodiments, the gene expression signature, or the subset thereof, of the present invention comprises or consists of at least 98%, at least 95%, at least 90%, at least 80%, at least 70%, at least 60%, at least 50%, at least 40%, at least 30%, at least 20%, at least 10%, at least 5% or at least 1% of the genes set forth in Table 1.

In embodiments, the one or more genes are upregulated.

In embodiments, the progression of Ulcerative Colitis is defined as subjects who require treatment that extends beyond 5 Aminosalicylates (5-ASA) and/or corticosteroid treatment. In embodiments, the progression of Ulcerative Colitis is defined as subjects who require treatment escalation to biologics which would include anti-TNFα targeting agents. In embodiments, the progression of Ulcerative Colitis is defined as subjects who require treatment that extends beyond 5 Aminosalicylates (5-ASA) and/or corticosteroid treatment and who require treatment escalation to biologics which would include anti-TNFα targeting agents. In embodiments, the progression of Ulcerative Colitis in a subject is from mild to moderate to severe.

In embodiments, the therapeutic agent is an anti-TNFα targeting agent. In embodiments, the anti-TNFα targeting agent is infliximab, adalimumab or golimumab.

According to a further aspect of the present invention, there is provided one or more biomarkers for use in vitro diagnosis of Ulcerative Colitis or for predicting the likelihood of disease progression of Ulcerative Colitis, wherein the biomarkers comprise or consist of the nucleic acid sequences of the mRNA sequences of the one or more genes listed in Table 1. In embodiments, the biomarkers comprise or consist of one or more of the nucleic acid sequences of SEQ ID NO:1 to 34 or functionally active fragments or variants thereof.

According to a further aspect of the present invention, there is provided one or more biomarkers for use in the treatment of Ulcerative Colitis, wherein the biomarker comprises or consists of one or more genes selected from the group listed in Table 1. In embodiments, the biomarker comprises or consists of the nucleic acid sequence of the mRNA sequence of the one or more genes listed in Table 1. In embodiments, the biomarkers comprise or consist of one or more of the nucleic acid sequences of SEQ ID NO:1 to 34 or functionally active fragments or variants thereof.

According to a further aspect of the present invention, there is provided use of the gene expression signature of the present invention for the isolation or development of a compound useful for therapy or prevention of Ulcerative Colitis or a related disease.

In embodiments of the above aspects of the invention, the gene expression signature or a subset thereof comprises or consists of one or more genes selected from the group comprising or consisting of carboxypeptidase E (*CPE*), WAP four-disulfide core domain 1 (*WFDC1*)*,*Calsequestrin 2 (*CASQ2*), LIM domain only 3 (*LMO3*)*,* Synemin (*SYNM*)*,* Actin Gamma 2, smooth muscle (*ACTG2*), Patched domain containing 1 (*PTCHD1*)*,* Heat Shock protein family B (small) member 7 (*HSPB7*), Filamin C (*FLNC*)*,* Carboxypeptidase X, M14 family member 2 (*CPXM2*)*,* Methionine sulfoxide reductase B3 (*MSRB3*)*,* Actin alpha 2, smooth muscle (*ACTA2*), Monoamine oxidase B (*MAOB*)*,* Nexilin F-actin binding protein (*NEXN*)*,* Destrin, actin depolymerizing factor (*DSTN*)*,* Prostaglandin-endoperoxide synthase 1 (*PTGS1*)*,* Kelch like family member 23 (*KLHL23*)*,* Rho GTPase activating protein 10 (*ARHGAP10*)*,* Mitogen-activated protein kinase kinase kinase 20 (*MAP3K20*)*,* Glutathione peroxidase 8 (GPX8), Tensin 1 (*TNS1*)*,* Slit guidance ligand 2 (*SLIT2*)*,* Tropomyosin 2 (*TPM2*)*,* Caldesmon 1 (*CALD1*)*,* Receptor activity modifying protein 1 *(RAMP1),* Transgelin (*TAGLN*)*,* Myosin Light chain 9 *(MYL9),* Microtubule associated protein 1A *(MAP1A),* Zinc finger and BTB domain containing 16 *(ZBTB16),* Secreted frizzled related protein 1 *(SFRP1),* Cyclase associated actin cytoskeleton regulatory protein 2 (CAP2), Myosin light chain kinase *(MYLK),* Myomesin 1 *(MYOM1)* and RAS oncogene family *(RAB23).*

In embodiments of the above aspects of the invention, the gene expression signature comprises or consists of the mRNA sequences of the one or more genes and the mRNA sequences comprise or consist of the nucleic acid sequences of SEQ ID NO:1 to SEQ ID NO:34 or functionally active fragments or variants thereof; or any combination thereof.

In embodiments of the above aspects of the invention, the gene expression signature comprises or consists of one or more genes selected from the group comprising or consisting of Carboxypeptidase E (*CPE*)*,* Glutathione Peroxidase 8 (*GPX8*) and Zinc Finger and BTB Domain Containing 16 (*ZBTB16*)*.* In embodiments, the gene expression signature comprises or consists of the mRNA sequences of the one or more genes and the mRNA sequences comprise or consist of the nucleic acid sequences of SEQ ID NO:1 or a functionally active fragment or variant thereof, SEQ ID NO:20 or a functionally active fragment or variant thereof or SEQ ID NO:29 or a functionally active fragment or variant thereof; or any combination of SEQ ID NO:1, SEQ ID NO:20 or SEQ ID NO:29 or a functionally active fragments or variants thereof.

In embodiments of the above aspects of the invention, the gene expression signature comprises or consists of one or more genes selected from the group comprising or consisting of Mitogen-activated protein kinase kinase kinase 20 (*MAP3K20*)*,* Rho GTPase activating protein 1 (*ARHGAP10*) and Prostaglandin-endoperoxide synthase 1 (*PTGS1*)*.* In embodiments, the gene expression signature comprises or consists of the mRNA sequences of the one or more genes and the mRNA sequences comprise or consist of the nucleic acid sequences of SEQ ID NO:19 or a functionally active fragment or variant thereof, SEQ ID NO:18 or a functionally active fragment or variant thereof or SEQ ID NO:16 or a functionally active fragment or variant thereof; or any combination of SEQ ID NO:19, SEQ ID NO:18 or SEQ ID NO:16 or a functionally active fragments or variants thereof.

In embodiments of the above aspects of the invention, the gene expression signature, or the subset thereof, of the present invention comprises or consists of at least 98%, at least 95%, at least 90%, at least 80%, at least 70%, at least 60%, at least 50%, at least 40%, at least 30%, at least 20%, at least 10%, at least 5% or at least 1% of the genes set forth in Table 1.

In embodiments of the above aspects of the invention, the one or more genes are upregulated.

In embodiments of the above aspects of the invention, the progression of Ulcerative Colitis is defined as subjects who require treatment that extends beyond 5 Aminosalicylates (5-ASA) and/or corticosteroid treatment. In embodiments, the progression of Ulcerative Colitis is defined as subjects who require treatment escalation to biologics which would include anti-TNFα targeting agents. In embodiments, the progression of Ulcerative Colitis is defined as subjects who require treatment that extends beyond 5 Aminosalicylates (5-ASA) and/or corticosteroid treatment and who require treatment escalation to biologics which would include anti-TNFα targeting agents. In embodiments, the progression of Ulcerative Colitis in a subject is from mild to moderate to severe.

In embodiments of the above aspects of the invention, the methods of the present invention use the gene expression signature or "panel of genes" (i.e. UC markers as disclosed herein) based on the presence or absence of their expression. In some embodiments, the signature of UC markers will include at least one, two, three, four, five, six, seven, eight, or up to thirty-four markers. In some embodiments, the signature of UC markers will include at least thirty-four markers.

In embodiments of the above aspects of the invention, the panel may include a UC marker that is upregulated in UC relative to a control. Such signatures or panels may be used to screen a mammalian subject for the differential expression of one or more UC markers in order to make a determination on whether UC is present in the subject.

In embodiments of the above aspects of the invention, the UC markers that make up the signature or panel of the present invention are selected from Table 1. In a preferred embodiment, the methods of diagnosing or detecting the presence of UC in a mammalian subject comprise determining a differential expression level of RNA transcripts or expression products thereof from a signature or panel of UC markers in a biological sample obtained from the subject relative to the level of expression in a control, wherein the differential level of expression is indicative of the presence of UC in the subject from which the test sample was obtained. The differential expression in the biological sample may be higher and/or lower relative to a control as discussed herein.

In embodiments of the above aspects of the invention, the present invention may be practiced using each gene listed in Table 1 separately as a diagnostic or prognostic marker or a few genes combined into a panel display format so that several genes may be detected for an overall pattern or listing of genes to increase reliability and efficiency. Furthermore, any of the genes identified in the present invention may be used individually or as a set of genes in any combination with any of the other genes that are recited herein. Alternatively, genes may be ranked according to their importance and weighted and a level of likelihood of Ulcerative Colitis disease progression may be assigned.

Standard biological therapy for moderate-severe cases of UC consists in the use of Infliximab (IFX), an anti-tumour necrosis factor α (anti-TNFα) targeting agent. However, approximately 40% of the patients do not respond to IFX. Following further extensive experimentation, the present inventors have surprisingly identified that silencing Tissue Factor Pathway Inhibitor 2 (TFPI2) can represent a novel therapeutic strategy to improve response of UC patients to IFX. TFPI2 has been shown to be elevated in UC patients who are non-responders to IFX, however for the first time the inventors demonstrate how TFPI2 functions as a master transcriptional regulator and regulates expression of genes underpinning non-response to IFX. Furthermore, the inventors' data unravels a novel immuneregulatory role of TFPI2 exemplified by its ability to upregulate pro-inflammatory cytokines, which are indeed key to inflammation in UC as well as response to biologic treatment. Taken together, the invention extends to the therapeutic potential of TFPI2, which when perturbed in UC patients will allow improved response to IFX as well as reduced inflammation.

According to a further aspect of the present invention, there is provided a Tissue Factor Pathway Inhibitor 2 (TFPI2) antagonist for use in the treatment and/or prophylaxis of Ulcerative Colitis (UC), wherein the use of TFPI2 results in an improved response of a subject to an Ulcerative Colitis therapeutic agent.

In embodiments, the Tissue Factor Pathway Inhibitor 2 (TFPI2) antagonist is any substance capable of blocking, attenuating, reducing, delaying or otherwise interfering with TFPI2 signalling and/or the interaction between TFPI2 and a cell surface receptor. In embodiments, the Tissue Factor Pathway Inhibitor 2 (TFPI2) antagonist is a small molecule, a protein, an antibody or fragment thereof, a soluble TFPI2 receptor based trap, or a nucleic acid, such as an antisense molecule or an siRNA. In embodiments, the Tissue Factor Pathway Inhibitor 2 (TFPI2) antagonist is a monoclonal antibody. In embodiments, the monoclonal antibody is Concizumab.

In embodiments, the therapeutic agent is an anti-tumour necrosis factor α (anti-TNFα) targeting agent. In embodiments, the anti-TNFα targeting agent is infliximab, adalimumab or golimumab. In embodiments, the anti-TNFα targeting agent is infliximab.

According to a further aspect of the present invention, there is provided a method for the treatment and/or prophylaxis of Ulcerative Colitis (UC), said method comprising the steps of:
(i) administering to a subject in need thereof a Tissue Factor Pathway Inhibitor 2 (TFPI2) antagonist; and
(ii) administering to a subject in need thereof a therapeutically effective amount of an Ulcerative Colitis therapeutic agent.

In embodiments, the Tissue Factor Pathway Inhibitor 2 (TFPI2) antagonist is any substance capable of blocking, attenuating, reducing, delaying or otherwise interfering with TFPI2 signalling and/or the interaction between TFPI2 and a cell surface receptor. In embodiments, the Tissue Factor Pathway Inhibitor 2 (TFPI2) antagonist is a small molecule, a protein, an antibody or fragment thereof, a soluble TFPI2 receptor based trap, or a nucleic acid, such as an antisense molecule or an siRNA. In embodiments, the Tissue Factor Pathway Inhibitor 2 (TFPI2) antagonist is a monoclonal antibody. In embodiments, the monoclonal antibody is Concizumab.

In embodiments, the therapeutic agent is an anti-tumour necrosis factor α (anti-TNFα) targeting agent. In embodiments, the anti-TNFα targeting agent is infliximab, adalimumab or golimumab. In embodiments, the anti-TNFα targeting agent is infliximab.

In embodiments, administering a Tissue Factor Pathway Inhibitor 2 (TFPI2) antagonist results in the subject having an improved response to the anti-TNFα targeting agent. In embodiments, administering a Tissue Factor Pathway Inhibitor 2 (TFPI2) antagonist results in the subject having an improved response to infliximab.

In embodiments, Tissue Factor Pathway Inhibitor 2 (TFPI2) is identified as SEQ ID NO:35.

According to a further aspect of the present invention, there is provided a composition for use in the treatment and/or prophylaxis of Ulcerative Colitis (UC), said composition comprising (i) a Tissue Factor Pathway Inhibitor 2 (TFPI2) antagonist; and (ii) a therapeutically effective amount of an Ulcerative Colitis therapeutic agent.

In embodiments, the Tissue Factor Pathway Inhibitor 2 (TFPI2) antagonist is any substance capable of blocking, attenuating, reducing, delaying or otherwise interfering with TFPI2 signalling and/or the interaction between TFPI2 and a cell surface receptor. In embodiments, the Tissue Factor Pathway Inhibitor 2 (TFPI2) antagonist is a small molecule, a protein, an antibody or fragment thereof, a soluble TFPI2 receptor based trap, or a nucleic acid, such as an antisense molecule or an siRNA. In embodiments, the Tissue Factor Pathway Inhibitor 2 (TFPI2) antagonist is a monoclonal antibody. In embodiments, the monoclonal antibody is Concizumab.

In embodiments, the therapeutic agent is an anti-tumour necrosis factor α (anti-TNFα) targeting agent. In embodiments, the anti-TNFα targeting agent is infliximab, adalimumab or golimumab. In embodiments, the anti-TNFα targeting agent is infliximab.

In embodiments, the use of the Tissue Factor Pathway Inhibitor 2 (TFPI2) antagonist results in the subject having an improved response to the anti-TNFα targeting agent. In embodiments, the use of the Tissue Factor Pathway Inhibitor 2 (TFPI2) antagonist results in the subject having an improved response to infliximab.

In embodiments, Tissue Factor Pathway Inhibitor 2 (TFPI2) is identified as SEQ ID NO:35.

According to a further aspect of the present invention, there is provided a pharmaceutical composition for use in the treatment and/or prophylaxis of Ulcerative Colitis (UC), said pharmaceutical composition comprising (i) a Tissue Factor Pathway Inhibitor 2 (TFPI2) antagonist; (ii) a therapeutically effective amount of an Ulcerative Colitis therapeutic agent; and (iii) a pharmaceutically acceptable excipient, diluent or carrier.

In embodiments, the Tissue Factor Pathway Inhibitor 2 (TFPI2) antagonist is any substance capable of blocking, attenuating, reducing, delaying or otherwise interfering with TFPI2 signalling and/or the interaction between TFPI2 and a cell surface receptor. In embodiments, the Tissue Factor Pathway Inhibitor 2 (TFPI2) antagonist is a small molecule, a protein, an antibody or fragment thereof, a soluble TFPI2 receptor based trap, or a nucleic acid, such as an antisense molecule or an siRNA. In embodiments, the Tissue Factor Pathway Inhibitor 2 (TFPI2) antagonist is a monoclonal antibody. In embodiments, the monoclonal antibody is Concizumab.

In embodiments, the therapeutic agent is an anti-tumour necrosis factor α (anti-TNFα) targeting agent. In embodiments, the anti-TNFα targeting agent is infliximab, adalimumab or golimumab. In embodiments, the anti-TNFα targeting agent is infliximab.

In embodiments, the use of the Tissue Factor Pathway Inhibitor 2 (TFPI2) antagonist results in the subject having an improved response to the anti-TNFα targeting agent. In embodiments, the use of the Tissue Factor Pathway Inhibitor 2 (TFPI2) antagonist results in the subject having an improved response to infliximab.

In embodiments, Tissue Factor Pathway Inhibitor 2 (TFPI2) is identified as SEQ ID NO:35.

In embodiments of the above aspects of the invention, the subject is a human. In embodiments, the subject is a human patient diagnosed with or at risk of developing Ulcerative Colitis. The subject may also be an Ulcerative Colitis patient who has received prior treatment for Ulcerative Colitis but is at risk of a recurrence of Ulcerative Colitis.

In embodiments of the above aspects of the invention, the biological sample obtained from the subject is a blood sample or a colonic tissue biopsy. In a preferred embodiment, the biopsy is a tissue selected from the group consisting of terminal ileum, the ascending colon, the descending colon, and the sigmoid colon. In other preferred embodiments, the biopsy is from an inflamed colonic area or from a non-inflamed colonic area. The inflamed colonic area may be acutely inflamed or chronically inflamed. In embodiments of the above aspects of the present invention, the biological sample is selected from the group consisting of tissue, blood, plasma, serum, stool, urine, urine supernatant, urine cell pellet, semen, colorectal secretions and colorectal cells. In embodiments, the biological sample of the subject is a colorectal tissue sample, preferably of a colorectal polyp, or a blood or serum sample taken from the circulatory system of the subject.

In embodiments of the above aspects of the present invention the subject is typically a human but also can be any mammal, including, but not limited to, a dog, cat, rabbit, cow, bird, rat, horse, pig, or monkey.

In embodiments of the above aspects of the present invention, the biological sample contains DNA or RNA from the subject. In embodiments, the biological sample comprises nucleic acids.

In embodiments of the above aspects of the present invention, the methods describes are in vitro methods.

### Description

The inventors of the present invention have surprisingly discovered molecular biomarkers that can predict disease progression of Ulcerative Colitis (UC) at an early stage i.e. prior to 5-ASA and corticosteroid treatment and therefore result in personalised treatment of patients and ultimately improve patient quality of life. The present invention provides a method for predicting the progression of UC in a subject and/or screening for an increased likelihood of progression of UC in a subject.

The invention disclosed herein provides methods and assays examining expression of one or more gene expression markers in a mammalian tissue or cell sample, wherein the expression of one or more such biomarkers is predictive of whether the mammalian subject from which the tissue or cell sample was taken is more likely to have UC that will progress into moderate or severe stage disease. In various embodiments of the invention, the methods and assays examine the expression of gene expression markers such as those listed in Table 1.

The particular clinical application of the present invention is to prioritise treatments for those patients identified as likely non-responders to the first line maintenance therapy 5-ASA who can then be moved onto more potent drugs earlier thereby improving the clinical outcome for the patient as well as generating savings for healthcare providers by reducing hospitalisations and surgery. The inventors have identified a novel gene expression signature consisting of 3460 genes or a subset thereof, which demonstrates the potential to predict disease progression in UC patients at a very early stage of their treatment regime. The method of the present invention comprises genetically testing a subject for the presence of one or more of the genes in the expression signature. The method is carried out *in vitro,* for example, in a laboratory, using a biological sample obtained from the subject. The present invention will, provide a much-needed early intervention for UC patients and by commencing early treatment improve overall patient outcome.

Currently, measurement of disease progression in UC patients is largely limited to measurement of disease activity/inflammation measured through markers such as C-reactive protein (CRP) and fecal calprotectin (FCP). While CRP, is a general marker of inflammation and therefore not specific to UC, FCP is regarded as a good measure of alteration in disease activity. However, more recent studies now show that high FCP levels are likely not a true indication of change in disease activity and rather due to other lesions like polyps^{[9]}. Therefore, caution is warranted to utilise these markers to determine treatment escalation. Moreover, variation in clinical set point cut-offs globally as well as modest ability of FCP to predict mucosal healing are the other limitations of this methodology ^{[10]}. As such, there are no current approaches that enable effective prediction of disease progression in UC which is marked by the need to escalate treatment beyond 5 ASA and corticosteroids or to escalate treatment to biologics which would include anti-TNFα targeting agents.

It is well established that gene expression alterations underpin pathogenesis of Ulcerative colitis. To that end for the first time, the present inventors have identified a gene expression signature consisting of 34 genes which are differentially expressed between UC progressors vs non-progressors. 34 genes are upregulated in UC progressor patients i.e. patients who require treatment escalation beyond 5 Aminosalicyclates (5 ASA) and corticosteroids. Interestingly, gene ontology as well as immune deconvolution reveals that a significant proportion of the genes from this signature are involved in tissue reorganization as well as immune-associated processes. Given the importance of immune dysregulation underlying UC progression, it very plausible that the gene expression signature or a subset thereof has the potential to predict disease progression at early onset of UC in patients.

To identify approaches to predict disease progression in UC patients, the inventors carried out detailed transcriptomic analysis for a total of 34 UC patients. To this purpose, the inventors defined disease progression as UC patients who required treatment escalation beyond 5 Aminosalicyclates (5 ASA) and corticosteroids. This definition allowed the inventors to stratify the 34 UC patients into two groups: progressors (n=25) and non-progressors (n=9). The gene signature consists of 34 genes. 3 key genes, CPE (Carboxypeptidase E, SEQ ID NO:1), GPX8 (Glutathione Peroxidase 8, SEQ ID NO:20) and ZBTB16 (Zinc Finger and BTB Domain Containing 16, SEQ ID NO:29), which were progressively upregulated in UC progressors, had important prognostic functions and are preliminarily the best predictors of progression.

RNA was extracted from histologically determined and demarcated inflamed and non-inflamed from each FFPE-derived patient tissue. RNA extraction was subsequently carried out for each sample followed by quantification, library prep and mRNA sequencing using the NovaSeq 5000 in a 2 x 75 bp manner. The sequencing data was analysed using the nextflow workflow (nf-core/RNAseq) followed by identification of differentially expressed loci between progressors vs non-progressors by using uninflamed data as background, thus ensuring that all differentially expressed genes (DEGs) are inflammation/disease specific.

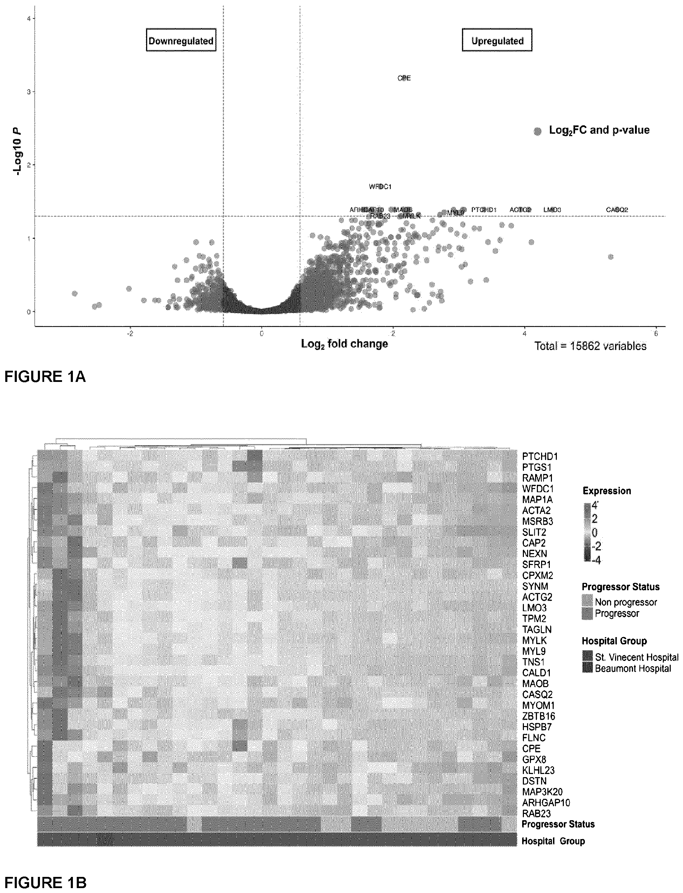

This analysis revealed a total of significant 34DEGs (adjusted p<0.05): 34 upregulated DEGs (log₂Fold change >0.58 in the UC progressor patients relative to non-progressors (Figure 1A). Furthermore, the inventors show that the 34 upregulated genes or a subset thereof, enabled a clear separation between progressor and non-progressor UC patients (Figure 1B).

While gene-ontology (GO) analysis revealed that the upregulated genes were enriched (Benjamini p-value<0.05) for GO terms such as *tissue morphogenesis, response to endogenous stimuli* and *developmental process* in general, pathway analysis interestingly showed that >10% of the upregulated genes were involved in: *R-HSA-1474244~Extracellular matrix organization* (Benjamini p=0.028) and *R-HSA-2173782-Binding and Uptake of Ligands by Scavenger Receptors* (Benjamini p=0.028). GO and pathway analysis for downregulated DEGs did not reveal any significant enrichment for GO terms and pathways.

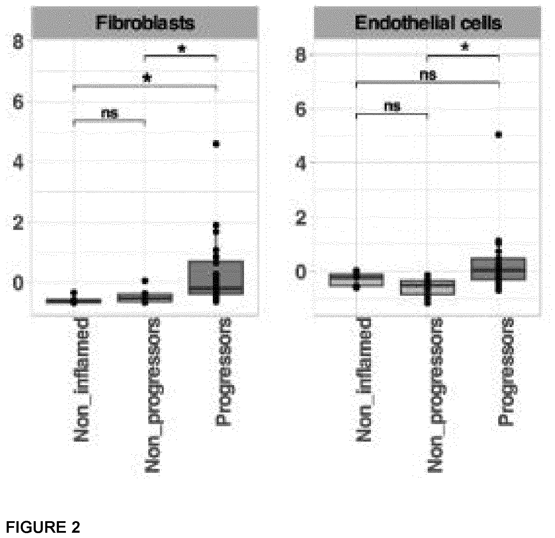

Given that inflammation is a key driver event underpinning UC disease progression, The Inventors next examined the mRNAseq data further to identify both immune and general cell types that might be different in abundance between progressor vs non-progressor UC patients. To this purpose, the inventors applied the mRNAseq data to MCP counter analysis that enables immune cell deconvolution. This analysis revealed that both endothelial cells and fibroblasts were significantly increased in the UC progressor patients compared to UC non-progressor and non-inflamed control tissue (Figure 2).

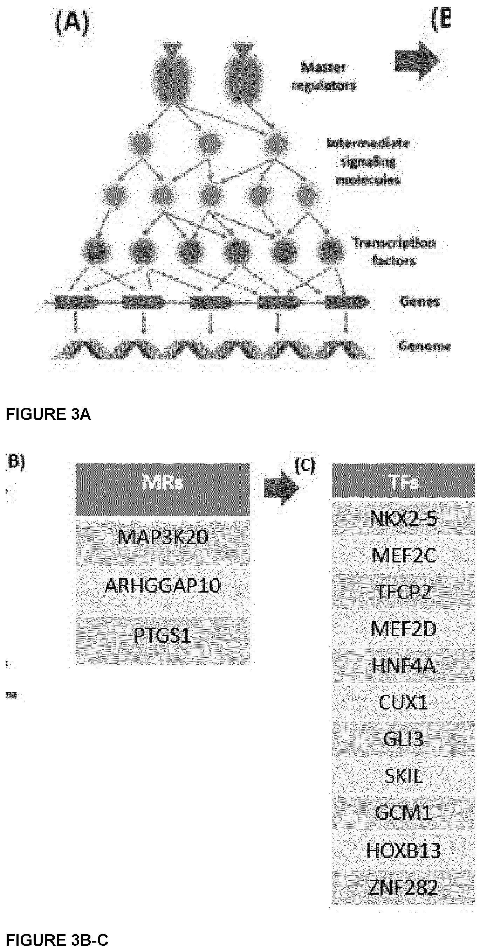

Both genetic and epigenetic factors likely drive disease progression mediated by global transcriptomic alterations in these patients. One such mechanism involves master transcription regulator (MR) mediated regulation of gene expression largely through transcription factors (TFs) (Figure 3A). To identify if indeed this was the case in the DEGs identified as upregulated in the progressor UC patients, the inventors applied the 34 upregulated genes to the GeneXplain platform. Briefly, this platform carries out an upstream analysis using DEGs allowing identification of transcription factor binding sites enriched with the DEGs. These binding sites are then assessed to generate composite modules which ultimately allows identification of intermediate molecules, followed by master transcriptional regulators. By applying the upregulated DEGs to this platform, the inventors identified 3 master regulators MAP3K20 (SEQ ID NO:19), ARHGAP10 (SEQ ID NO:18), PTGS1 (SEQ ID NO:16) (Figure 3B) which regulate gene expression through 11 distinctive TFs (Figure 3C). Additionally, the inventors evaluated the top three TFs (NKX2.-5, MEF2C, TFCP2) which are controlled by the top three MTRs (MAP3K20, ARHGAP10, PTGS1) to examine what genes from the gene signature list they are directly controlling. It was found that NKX2-5 controlled the highest number of genes, with a total of 31, followed by TFCP2 controlling 18 and lastly MEF2C controlling 5 of the 34 upregulated DEGs (Table 2). Importantly, the inventors identified that 4 (SYNM, WFDC1, MAP3K20, CASQ2) out of the 34 upregulated genes were commonly regulated by these 3 MRs and the top 3 TFs (Table 3).

**Table 2: List of genes controlled by the top three TFs. The differentially expressed genes (DEGs) were filtered using the score values assigned to each gene by Genome Enhancer. Of the upregulated genes, 18 are regulated by TFCP2, 6 by MEF2C, and31 by NKX2_5 transcription factors.**

| *The top three transcription factors identified as regulators of upregulated genes in progressors* | | |
|---|---|---|
| **TFCP2 TFs genes** | **MEF2C TFs genes** | **NKX2_5 TFs genes** |
| HSPB7 | ARHGAP10 | HSPB7 |
| DSTN | MYOM1 | DSTN |
| RAMP1 | SYNM | ARHGAP10 |
| MYLK | WFDC1 | RAMP1 |
| SYNM | MAP3K20 | MYLK |
| WFDC1 | CASQ2 | MYOM1 |
| FLNC | | NEXN |
| MAOB | | SYNM |
| TPM2 | | WFDC1 |
| MAP3K20 | | MSRB3 |
| MAP1A | | FLNC |
| ZBTB16 | | MAOB |
| TAGLN | | SFRP1 |
| CASQ2 | | CPXM2 |
| TNS1 | | CAP2 |
| CPE | | TPM2 |
| ACTA2 | | MAP3K20 |
| RAB23 | | ZBTB16 |
| | | PTGS1 |
| | | KLHL23 |
| | | LMO3 |
| | | ACTG2 |
| | | TAGLN |
| | | CALD1 |
| | | CASQ2 |
| | | TNS1 |
| | | PTCHD1 |
| | | CPE |
| | | SLIT2 |
| | | MYL9 |
| | | ACTA2 |

**Table 3: 4 (SYNM, WFDC1, MAP3K20, CASQ2) out of the 34 upregulated genes were commonly regulated by these 3 MRs and the top 3 TFs.**

| ***Master regulators*** | ***Transcription factors (TFs)*** | ***Regulatory score (TFs)*** | ***Overlapping genes from*** |
|---|---|---|---|
| *MAP3K20* | NKX2-5 | 1.376623377 | SYNM |
| *ARHGAP10* | MEF2C | 1.26984127 | WFDC1 |
| *PTGS1* | TFCP2 | 1.220779221 | MAP3K20 |
| | | | CASQ2 |

Thus, for the first time, the inventors have identified a gene expression signature comprising or consisting of 34 genes or a subset thereof, which are uniquely modulated in the UC progressors patient group and therefore likely to act as predictive biomarkers of disease progression in UC patients. The present invention provides a diagnostic assay based on the gene expression signature comprising or consisting of one or more of the 34 genes to predict disease progression in UC patients.

Moreover, the inventors also demonstrate the potential regulation of these genes mediated by a complex signalling network regulated by MRs through TFs.

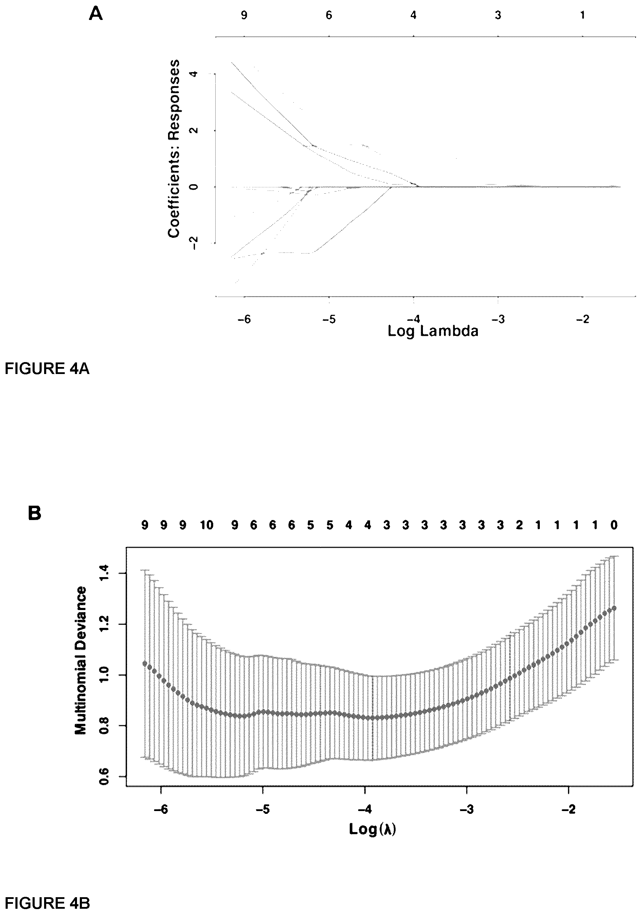

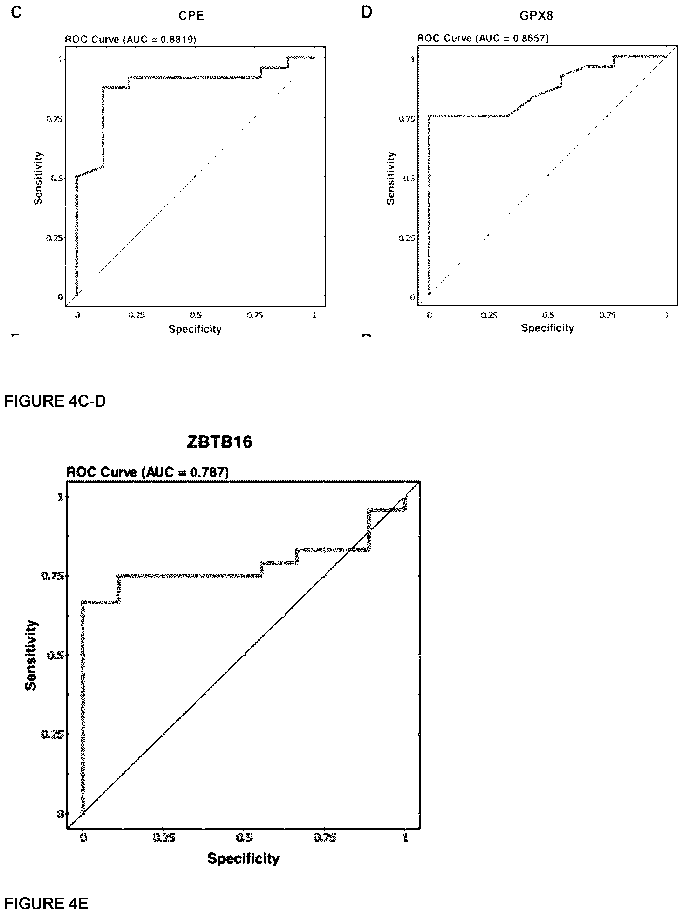

The inventors show that 3 key specific genes from the 34 upregulated genes namely: *CPE (SEQ ID NO:1), GPX8* (SEQ ID N:20) and *ZBTB16* (SEQ ID NO:29) (Figure 4A-B) have significant potential to predict disease progression in the discovery cohort, using Least Absolute Shrinkage and Selection Operator (LASSO) analysis. They sought to evaluate the effectiveness of the three predictors and the optimal cut-off value, by generating a Receiver operating characteristic curve (ROC curve) and deducing the area under the curve(AUC). This analysis identified high AUC values for each of the three genes: *CPE* (AUC = 0.88), *GPX8* (AUC = 0.87) and *ZBTB16* (AUC = 0.79). The ROC curve, AUC and boxplots of gene expression results cumulatively suggest that these genes have an excellent specificity and specificity to predict disease progression in UC patients (Figure 4C-E).

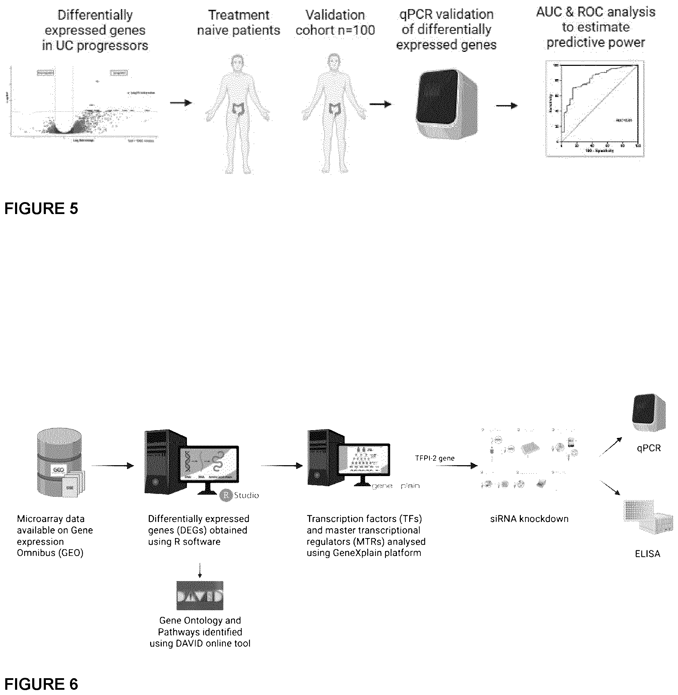

In order to further validate the predictive potential of this signature, the inventors have further examined the expression status of the gene signature in an independent cohort of UC patients as well as treatment naive UC patients using qPCR (Figure 5). Ultimately, the inventors have ascertained the predictive potential of the signature partially and/or in its entirety. The inventors have also assessed if these genes are altered in UC patients prior to any treatment and therefore could be used as a biomarker for these patients at disease initiation, therefore significantly strengthening the clinical utility of this signature.

The subject invention also provides an array of probe nucleic acids immobilized on a solid support, said array comprising: a plurality of probe nucleic acid compositions, wherein each probe nucleic acid composition is specific for a gene of the gene expression signature of the present invention, where in certain embodiments said array further comprises at least one control nucleic acid composition.

The subject invention also provides a kit for use in determining the phenotype of a source of a nucleic acid sample, said kit comprising: at least one of: (a) an array as described above; or (b) a collection of gene specific primers as described above. The kit may further comprise a software package for data analysis of expression profiles.

The term expression profile is used broadly to include a genomic expression profile, e.g., an expression profile of mRNAs, or a proteomic expression profile, e.g., an expression profile of one or more different proteins. Profiles may be generated by any convenient means for determining differential gene expression between two samples, e.g. quantitative hybridization of mRNA, labelled mRNA, amplified mRNA, cRNA, etc., quantitative PCR, ELISA for protein quantitation, and the like. For quantitative PCR analysis, the subject invention provides a collection of gene specific primers, said collection comprising: gene specific primers specific for at least about 10, at least about 20, at least about 30, at least about 40, at least about 50, at least about 60 of the genes of the gene expression signature of the present invention or at least the 34 upregulated genes of the gene expression signature of the present invention, where in certain embodiments said collection comprises as many gene specific primers as is necessary.

A subject or patient sample, e.g., cells or collections thereof, e.g., tissues or blood, is assayed. Samples are collected by any convenient method, as known in the art. Additionally, cells may be collected and tested to determine the relative effectiveness of a therapy in causing differential death between normal and diseased cells. Genes/proteins of interest are genes/proteins that are found to be predictive, including the genes/proteins provided in the gene expression profile of the present invention, where the expression profile may include expression data for any one of or all of the listed genes/proteins.

In certain embodiments, the expression profile obtained is a genomic or nucleic acid expression profile, where the amount or level of one or more nucleic acids in the sample is determined. In these embodiments, the sample that is assayed to generate the expression profile employed in the diagnostic methods is one that is a nucleic acid sample. The nucleic acid sample includes a plurality or population of distinct nucleic acids that includes the expression information of the phenotype determinative genes of interest of the cell or tissue being diagnosed. The nucleic acid may include RNA or DNA nucleic acids, e.g., mRNA, cDNA etc., so long as the sample retains the expression information of the host cell or tissue from which it is obtained.

The sample may be prepared in a number of different ways, as is known in the art, e.g., by mRNA isolation from a cell, where the isolated mRNA is used as is, amplified, employed to prepare cDNA etc., as is known in the differential expression art. The sample is typically prepared from cells from tissue or blood harvested from a subject to be diagnosed, using standard protocols, where cell types or tissues from which such nucleic acids may be generated include any tissue in which the expression pattern of the to be determined phenotype exists. Cells may be cultured prior to analysis.

The expression profile of the gene expression signature of the present invention may be generated from the initial nucleic acid sample using any convenient protocol. While a variety of different manners of generating expression profiles are known, such as those employed in the field of differential gene expression analysis, one representative and convenient type of protocol for generating expression profiles is array based gene expression profile generation protocols. Such applications are hybridization assays in which a nucleic acid that displays "probe" nucleic acids for each of the genes to be assayed/profiled in the profile to be generated is employed. In these assays, a sample of target nucleic acids is first prepared from the initial nucleic acid sample being assayed, where preparation may include labelling of the target nucleic acids with a label, e.g., a member of signal producing system. Following target nucleic acid sample preparation, the sample is contacted with the array under hybridization conditions, whereby complexes are formed between target nucleic acids that are complementary to probe sequences attached to the array surface. The presence of hybridized complexes is then detected, either qualitatively or quantitatively.

Specific hybridization technology which may be practiced to generate the expression profiles employed in the subject methods includes the technology described in the art. In these methods, an array of "probe" nucleic acids that includes a probe for each of the phenotype determinative genes whose expression is being assayed is contacted with target nucleic acids as described above. Contact is carried out under hybridization conditions, e.g., stringent hybridization conditions as described above, and unbound nucleic acid is then removed. The resultant pattern of hybridized nucleic acid provides information regarding expression for each of the genes that have been probed, where the expression information is in terms of whether or not the gene is expressed and, typically, at what level, where the expression data, i.e., expression profile, may be both qualitative and quantitative.

Alternatively, non-array-based methods for quantitating the levels of one or more nucleic acids in a sample may be employed, including quantitative PCR, and the like.

Where the expression profile is a protein expression profile, any convenient protein quantitation protocol may be employed, where the levels of one or more proteins in the assayed sample are determined. Representative methods include, but are not limited to; proteomic arrays, flow cytometry, standard immunoassays, etc.

Following obtainment of the expression profile from the sample being assayed, the expression profile is compared with a reference or control profile to make a diagnosis. A reference or control profile is provided, or may be obtained by empirical methods from samples. In certain embodiments, the obtained expression profile is compared to a single reference/control profile to obtain information regarding the phenotype of the cell/tissue being assayed. In yet other embodiments, the obtained expression profile is compared to two or more different reference/control profiles to obtain more in depth information regarding the phenotype of the assayed cell/tissue. For example, the obtained expression profile may be compared to a positive and negative reference profile to obtain confirmed information regarding whether the cell/tissue has the phenotype of interest.

The difference values, i.e. the difference in expression may be performed using any convenient methodology, where a variety of methodologies are known to those of skill in the array art, e.g., by comparing digital images of the expression profiles, by comparing databases of expression data, etc. A statistical analysis step is then performed to obtain the weighted contribution of the set of predictive genes.

In many embodiments, the above-obtained information about the cell/tissue being assayed is employed to predict whether a host, subject or patient should be treated with a therapy of interest and to optimize the dose therein. In embodiments the therapy is a UC therapy other than 5-ASA treatment or corticosteroids. In embodiments the therapy is a UC therapy other than 5-ASA treatment and corticosteroids.

The inventors identified Infliximab (IFX) as a commonly used biological therapy for moderate-severe cases of Ulcerative Colitis (UC). Infliximab (IFX) is an anti-tumour necrosis factor α (anti-TNFα) targeting agent. However, approximately 40% of the patients do not respond to IFX. It is difficult to predict the 40% of patients who will not respond, which renders colectomy inevitable in a substantial proportion of these patients. The disparity of treatment response is often due to the dynamics of UC pathophysiology underpinned by interaction of various factors such as genetic predispositions, environmental factors, aberrant immune responses, inflammatory response and microbial infection. It is therefore crucial to predict and distinguish between IFX non-responders patients and patients that respond effectively, to ultimately increase clinical benefits and avoid potential adverse effect. While both genetic and epigenetic factors have been reported to impact UC pathogenesis, their role in regulating treatment response remains unclear. Identifying regulatory factors and the precise mechanism by which these factors mediate regulatory control of these genes is of great importance, which can serve as potential therapeutic targets whose perturbation may improve patient response and outcomes by increasing sensitivity to IFX. Several studies to date have used microarray data to identify gene expression profiles that can predict the response to anti- TNFα antibodies in IBD patients.

In order to understand why some patients do not respond to IFX, the inventors performed a bioinformatic analysis to identify elements called master transcriptional regulators (MTRs) controlling gene expression in UC patients resistant to IFX followed by studies conducted in cell lines to establish their functional role in UC pathogenesis. Using public datasets, the inventors identified the differentially expressed genes (DEGs) and the respective transcription factors (TFs) enriched for the DEG's. This was followed by an upstream analysis to identify MTR's which regulate expression of these genes through the TFs. The Inventors next explored the role of these MTRs in UC silencing target genes in a novel IBD-like *in vitro* system comprising of normal rectal epithelial cells treated with TNFα. The inventors showed that TFPI2 (tissue factor pathway inhibitor 2) was significantly upregulated in an IBD-like in vitro model. Silencing of TFPI2 in this model resulted in a significant decrease in gene expression, all of which were predicted to be upregulated by TFPI2 from their bioinformatic analysis. Intriguingly, the inventors also showed that downregulation of TFPI2 resulted in significant reduction of inflammation. The inventors, for the first time, identify key MTRs such as TFPI2 which acts as a master regulator of genes whose upregulation drives poor response to IFX. Therefore, TFPI2 can be identified as a potential therapeutic target, whose perturbation in UC patient would likely result in improved response to IFX.

Previous reports have demonstrated that TFPI2 levels are elevated in UC patients who are non-responders to IFX, however no study to date has demonstrated the functional role of TFPI2 in IBD and thereby supporting its potential as a therapeutic target in UC patients. To that end, using an *in vitro* system, the inventors show that of the MTRs identified in their *in-silico* analysis, only TFPI2 (identified as MTR of upregulated genes in IFX non-responders) was significantly upregulated post-TNFα treatment. Following the knockdown of TFPI2 in their in vitro IBD-like model, the inventors found TFPI2 to be significantly decreasing *TNFAIP3, FCGR3B, TNFAIP6, CXCL5* and *BIRC3* expression in cells stimulated with TNFα when compared to control (scrambled control). When TFPI2 is knocked down it resulted in decreased levels of proinflammatory cytokines, which indicates that TFPI2 has a proinflammatory function in non-responders to IFX. In the inventor's study knocking down TFPI2 revealed significant reduction in pro-inflammatory cytokines levels including IL6, IL1β, IL8, and IFNy. These cytokines are well known drivers of inflammation and hence have significant implications on the pathogenesis of UC and the effectiveness on IFX, which specifically targets proinflammatory cytokines. In fact, excessive production of these cytokines is considered a critical factor in the development of UC. Without being bound by theory, the inventors submit that by reducing the levels of inflammatory response after knockdown of TFPI2, this MTR could act as therapeutic target in IBD, potentially enhancing the efficacy of IFX treatment. Therefore, from a functional perspective, the inventors' data reveals a novel immune-modulatory role of TFPI2. Ultimately, these novel functions of TFPI2 in UC, rationalises its importance as a therapeutic target, which when modulated likely leads to reduction in inflammation as well as increased response to IFX in patients with UC.

The inventors used a microarray meta-analysis approach to predict IFX non-responders from responders in UC. They gathered all publicly available microarray datasets involving UC patients treated with IFX (Figure 6) from the Gene Expression Omnibus (GEO). Functional validation of the outputs of this in silico analysis, using an IBD-like *in vitro* model demonstrates the potential of TFPI2 as a potential therapeutic target which when perturbed leads to reduced significant downregulation of inflammatory cytokines and thereby likely increases sensitivity of patients to IFX.

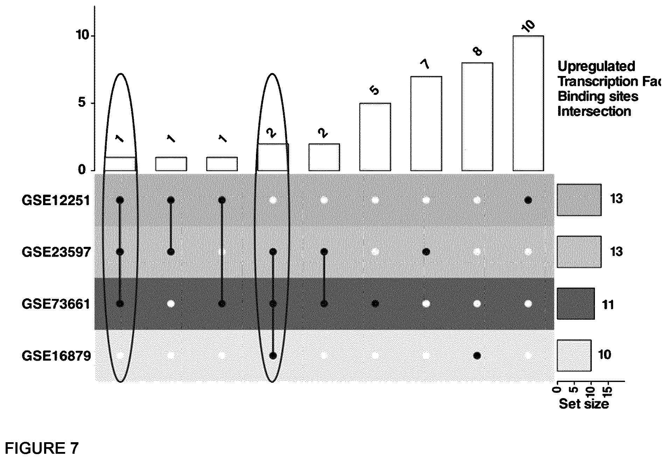

The inventors performed a robust *in-silico* analysis to identify master transcriptional regulators (MTRs) which regulate gene expression changes in IFX non-responsive UC patients, followed by perturbation *in vitro* to establish their functional role in UC pathogenesis. Differentially expressed genes (DEGs) identified from four independent public datasets (GSE12251, GSE73661, GSE23597, and GSE16879) were applied to the GeneXplain platform to identify transcription factors (TFs) enriched for the DEG's. This was followed by an upstream analysis to identify MTR's which regulate expression of these genes through the TFs. The inventors next explored the role of these MTRs in driving UC-associated phenotype, using siRNA-mediated knock-down and RNA sequencing in a novel IBD-like *in vitro* system comprising of normal rectal epithelial cells treated with TNFα. Over 200 TFs were identified in both upregulated and downregulated genes in IFX non-responders across all data sets (Table 4). RELA, NANOG, FOSJUN were the top 3 TF' enriched for upregulated genes in three out of the four datasets (Figure 7). Using the TF data, the upstream analysis identified CXCL8, SELE, PTGS2, FCGR3, TFPI2 as the top five MTRs (Table 5). The mRNA and the peptide sequences for the top 5 MTRs are listed as SEQ ID NOs:36-53 in Table 5.

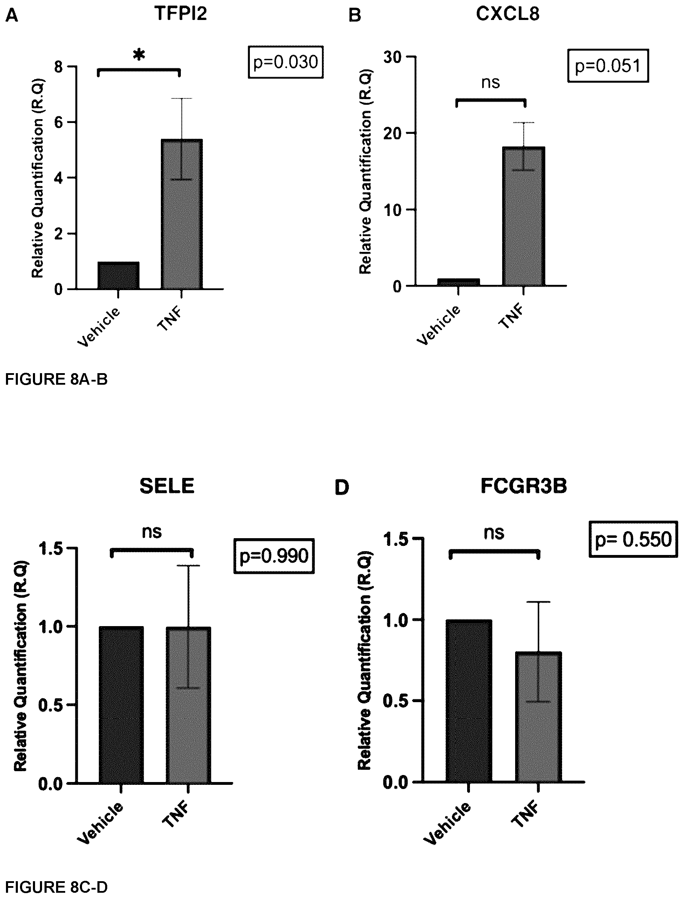

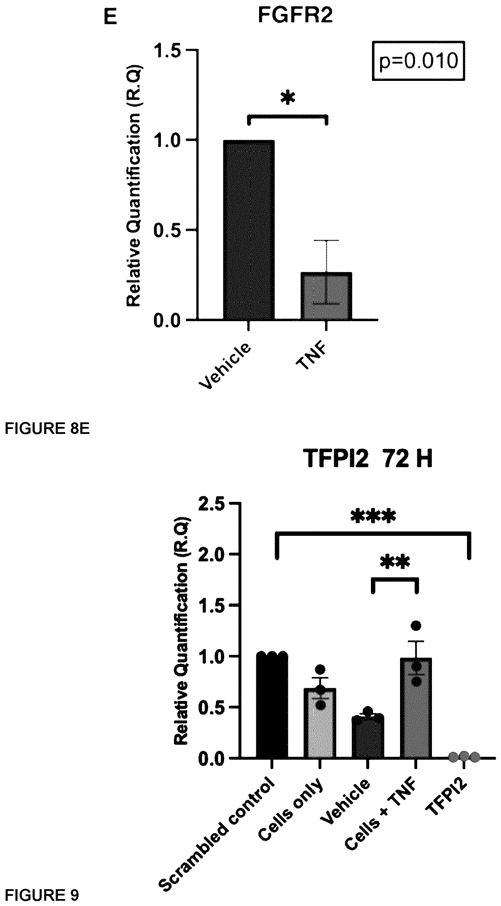

Next, in order to functionally assess the role of the MTR's in driving an IBD-associated phenotype, the inventors sought to ascertain the mRNA levels of these MTRs (*CXCL8, SELE, PTGS2, FCGR3, TFPI2*) in their in vitro IBD-like model system (CRL1831 + 20 ng TNFA α). As shown in Figure 8, TFPI2 showed significant upregulation (p=0.03), while FGFR2 levels were significantly downregulated (p=0.01) following TNFα stimulation for 24 hrs relative to vehicle treated CRL1831 cells. mRNA expression levels of all remaining MTR's were insignificantly altered following TNFα stimulation. TFPI2 is part of the Kunitz-type serine protease inhibitor family. The gene sequence for TFPI2 is identified as SEQ ID NO:35. TFPI2 has been reported to play essential roles in pathogenesis and anti- TNFα therapy resistance in IBD. In addition, publicly available datasets have shown elevated levels of TFPI2 in non-responders with IBD. However, the functional role of TFPI2 as a master transcriptional regulator of gene underpinning non-response to IFX is still largely unknown. Furthermore, no studies to date have demonstrated how silencing TFPI2 can represent a novel therapeutic strategy to improve response of UC patients to IFX. As aforementioned, the inventors found TFPI2 to be upregulated in non-responders in silico analysis as well as a significant increase in expression of TFPI2 following TNFα stimulation in CRL1831 cells. Given that increased level of TFPI2 in their IBD-like *in vitro* model is consistent with their *in-silico* analysis, together with the fact that functional role of TFPI2 is still largely unknown, the inventors examined the functional impact of this MTR on UC-associated phenotype.

**Table 4: List of top 20 TFs obtained for upregulated and downregulated genes. From the upregulated genes, 343 TFs were commonly significant, while 218 TFs were commonly significant in downregulated genes. TFs were ordered according to p-value then intersected.**

| **Upregulated TFs** | | | **Downregulated TFs** | | |
|---|---|---|---|---|---|
| NFYA | ETV7 | SOX30 | PAX6 | RUNX3 | KLF6 |
| SOX8 | BRCA1 | FOXP3 | TFAP2B | HSF2 | MEF2A |
| ETV1 | NR1H4 | NFE2L3 | TFAP2D | POU2F2 | TFE3 |
| PAX6 | ISL1 | MXD1 | PAX7 | ZIC2 | BCL3 |
| NFIX | SNAI2 | GSC2 | NR1H4 | FOXP3 | TCF3 |
| PAX7 | RUNX3 | DLX3 | ISL1 | GSC2 | NFATC3 |
| HSF2 | POU2F2 | SNAI2 | POU1F1 | | |

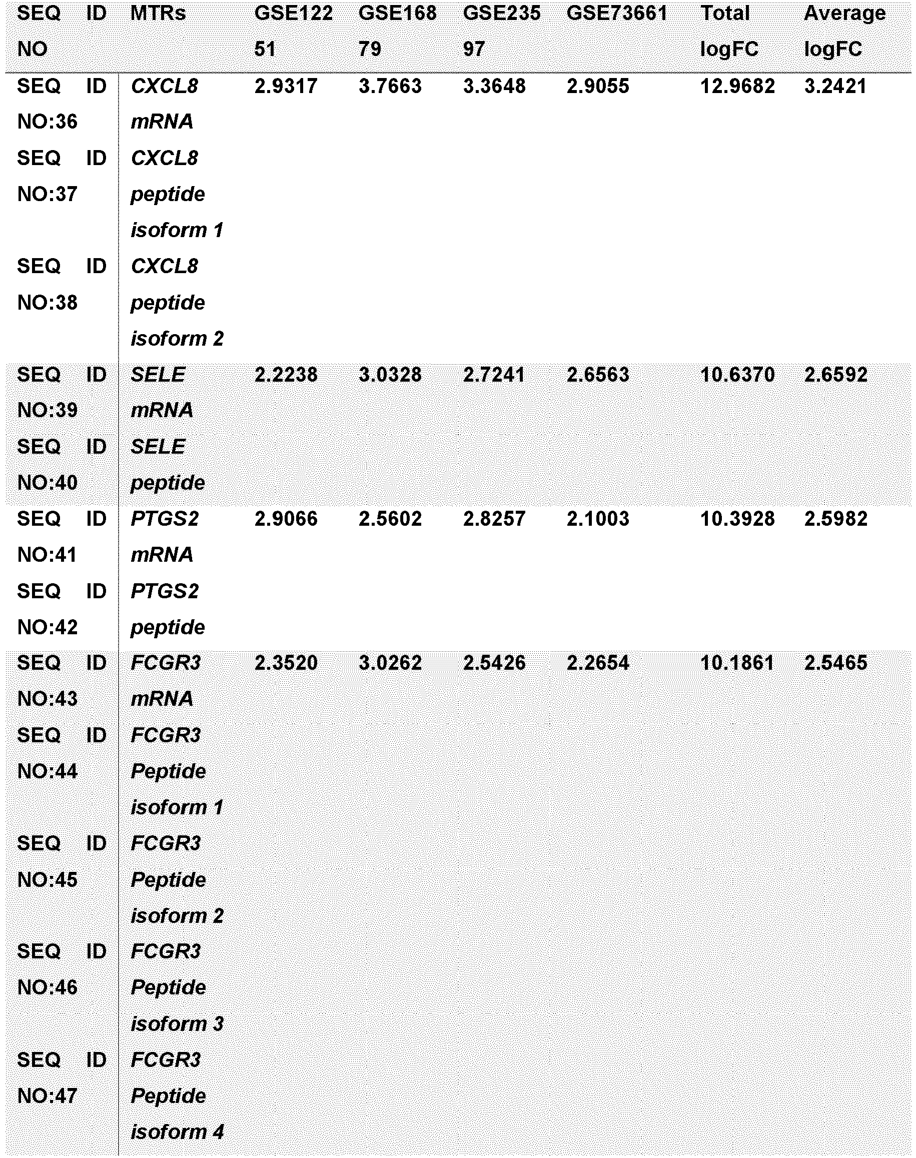

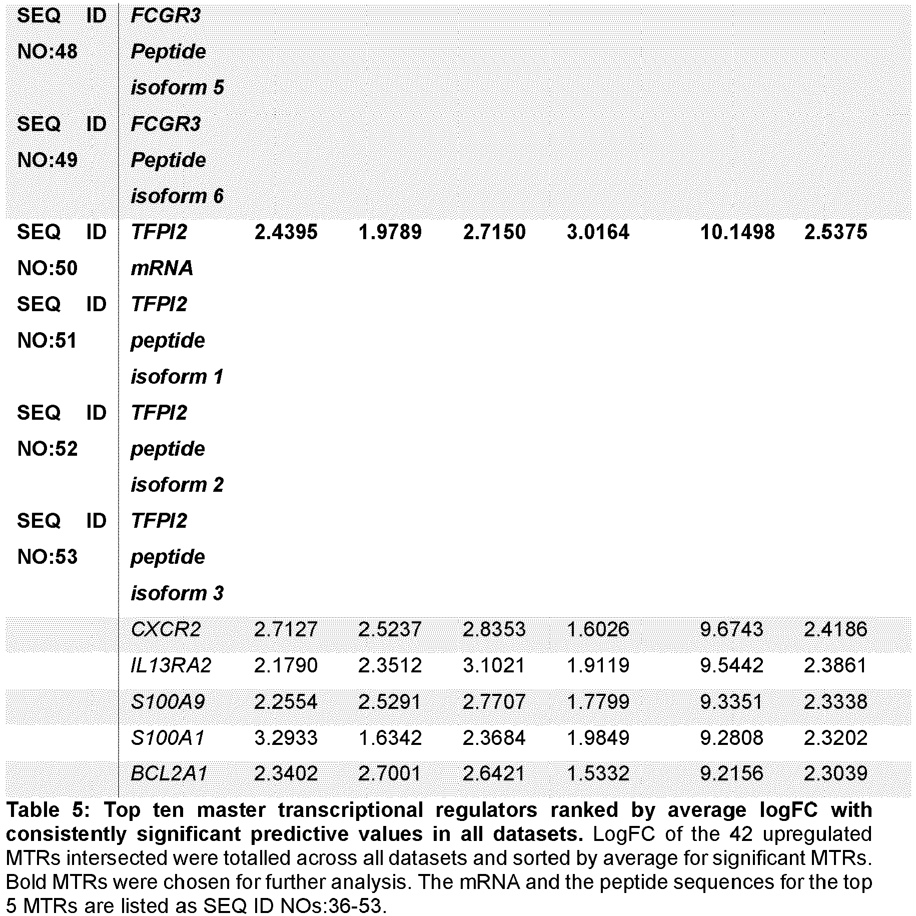

To next ascertain the precise functional role of TFPI2 in UC, the inventors performed a siRNA-mediated knock-down of TFPI2 in the CRL1831 cells + TNFα. RT- qPCR analysis revealed significantly decreased mRNA expression levels of TFPI2 in knockdown cells compared with scrambled control (Figure 9).

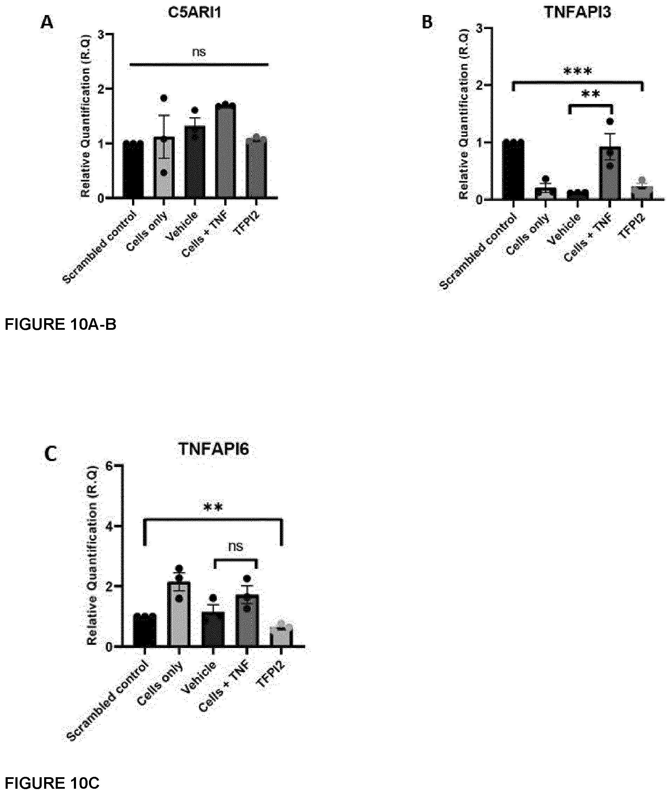

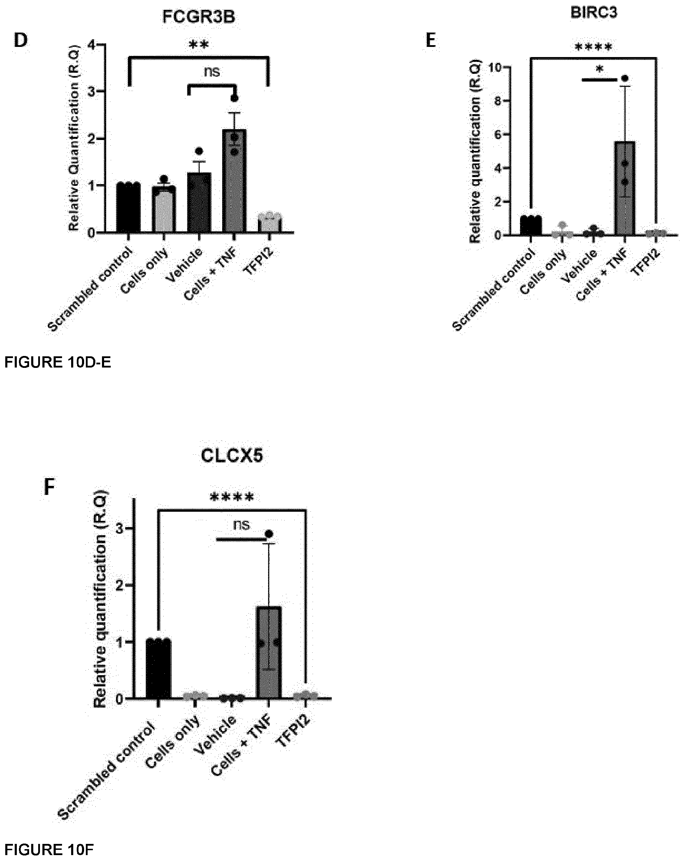

To examine the impact of TFPI2 knockdown on gene expression, RT- qPCR was carried out on the identified target gene list which appeared to be controlled by the top 5 MTRs including TFPI2 (Table 6). The inventors examined expression of three genes - *TNFAIP6, CXCL8,* and *C5AR1* which were identified to be upregulated and commonly regulated by the top three TFs namely, RELA, NANOG and FOSJUN. In addition, the inventors also evaluated expression of *TNFAIP3, FCGR3B* as well as *CXCL5, BIRC3* identified to be regulated by NANOG and FOSJUN and RELA and NANOG respectively. siRNA-mediated knock-down of TFPI2 *in vitro* resulted in a significant decrease in genes including *TNFAIP6* (p < 0.01), *TNFAIP3* (p < 0.001), *FCGR3B* (p < 0.01), *BIRC3* (p < 0.001), and *CXCL5* (p < 0.001), all of which were predicted in silico to be upregulated by TFPI2 during the upstream analysis (Figure 10).

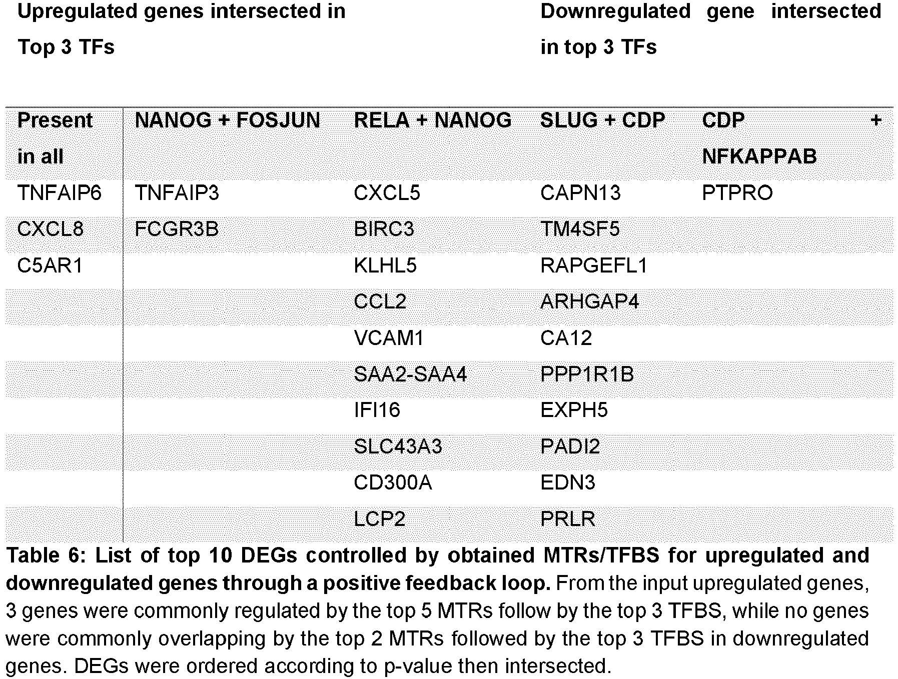

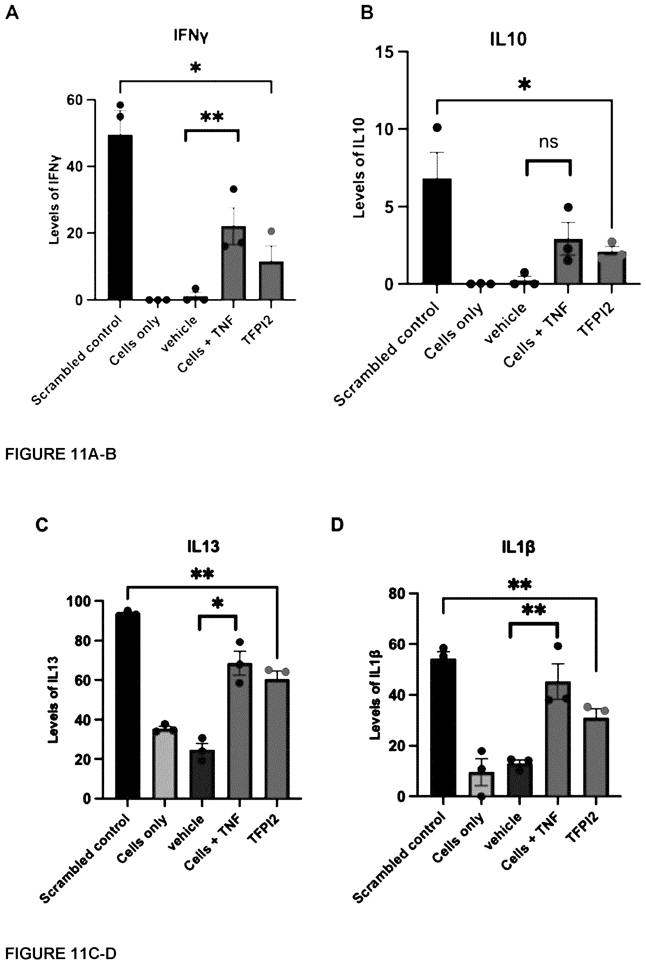

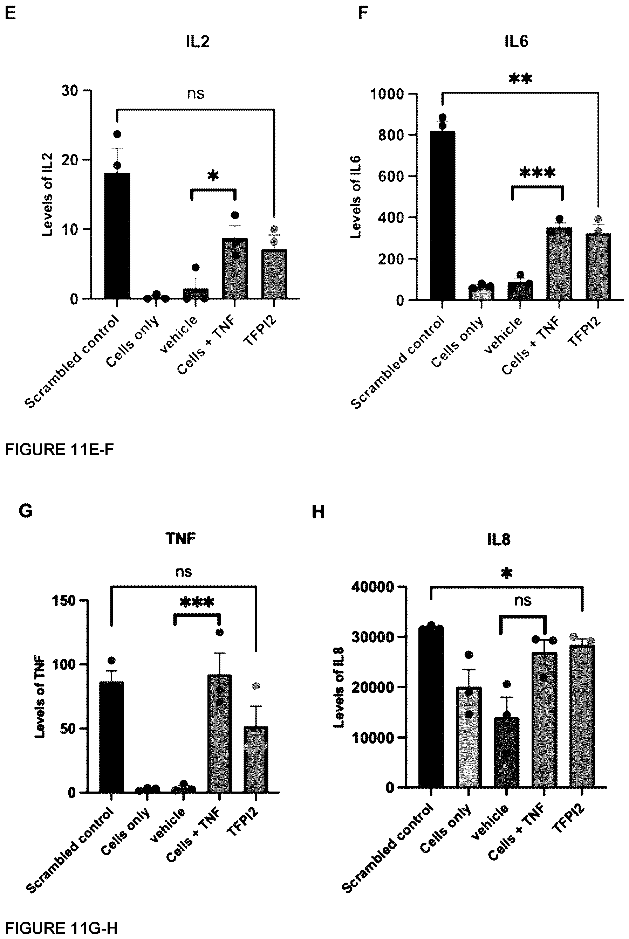

Enrichment analyses revealed that TFPI2 plays a vital role in numerous immunerelated biological processes and signalling pathways. These include the *regulation of immune response system process, innate immune system, cytokine signalling in the immune system,* TNFα *signalling pathways,* and *cytokine-cytokine receptor interaction.* This strongly suggests that TFPI2 plays a critical role in the immune response of UC. Hence, we evaluated the regulation of cytokine expression after TFPI2 knockdown, using the Mesoscale MultiSPOT platform. Levels of cytokines were significantly elevated in cells stimulated with TNFα when compared with vehicles (controls). After silencing TFPI2, levels of IL1β, IFNY, IL10, IL13, IL6 and IL8 were lower compared to the vehicles (Figure 11). However, despite our efforts to measure the levels of IL4 and IL12p70, the concentrations of these proteins exceeded the range of the standard curve. As a result, we were unable to accurately quantify them in our samples. Furthermore, the results obtained from ELISA showed that the stimulation with TNFα in CRL1831 has an impact on increasing cytokines levels, confirming that our *in vitro* system mimic the inflammatory environment IBD-like.

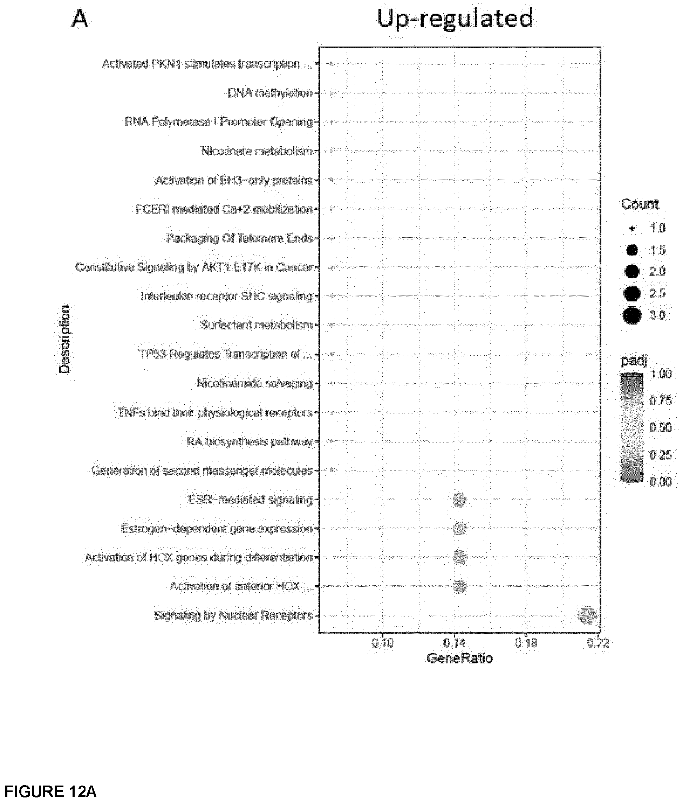

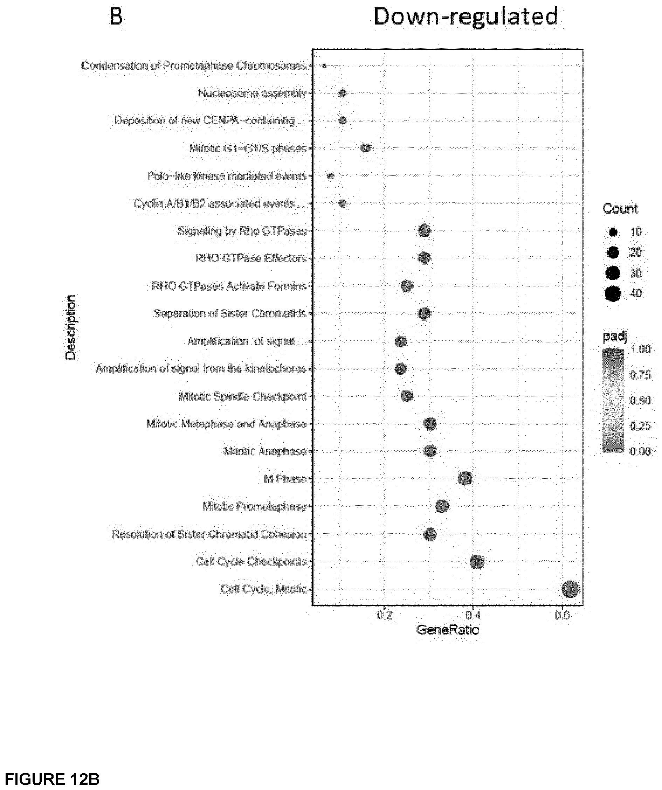

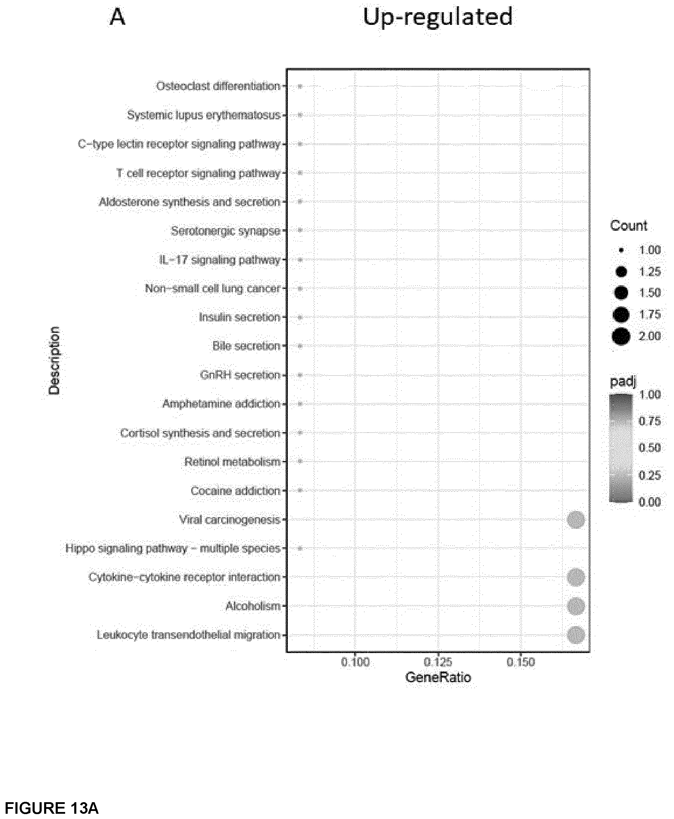

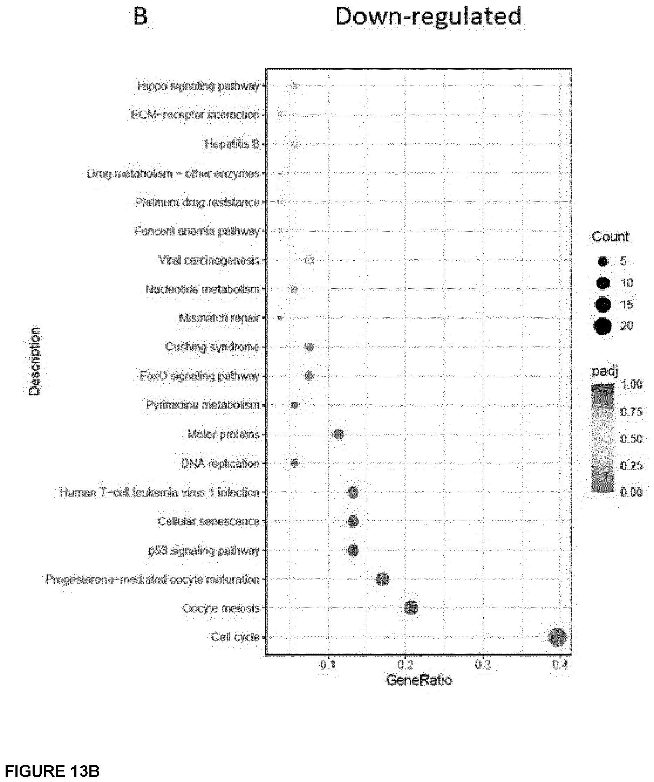

Results from RNA sequencing revealed a number of pathways significantly up and downregulated after TFPI2 knockdown. Reactome analysis showed an upregulation in some immune pathways including *TNFs binding their physiological receptors* and *interleukin receptor SHC signalling* (Figure 12A), while downregulation of pathways associated with cell cycle regulation (i.e *cell cycle mitotic, cell cycle checkpoints, M phase)* and chromosome stability (i.e *nucleosome assembly, condensation of promethaphase chromosomes, separation of sister chromatid is*) was observed (Figure 12B). Different biological pathways based on the Kyoto Encyclopedia of genes and genomes (KEGG) were found to be differentially regulated after TFPI2 knockdown. Specifically, *upregulation of T cell receptor signalling pathways, IL17 signalling pathway* and *cytokines-cytokines interaction* was observed (Figure 13A), while downregulation of *cell cycle, cellular senescence, DNA replication* and *p53 signalling pathways* was found (Figure 13B). These results indicate that TFPI2 knockdown has an impact on the immune response and the inflammation signalling. Cumulatively, results from qPCR and ELISA support the hypothesis that the knockdown of TFPI2 alter the expression of downstream genes and have an impact on inflammatory molecules levels. Our findings imply that TFPI2 may be relevant to the pathogenesis and progression of UC and can act as a potential therapeutic target, whose perturbation in UC patient would likely result in improved response to IFX.

### Definitions

Unless otherwise defined, all technical and scientific terms used herein have the meaning commonly understood by a person who is skilled in the art in the field of the present invention.

The terms 'progression' or 'progression of Ulcerative Colitis' or 'disease progression' as used herein can be determined as subjects who have Ulcerative Colitis who require treatment that extends beyond 5 Aminosalicylates (5 ASA) and/or corticosteroid treatment. Disease progression means that these UC patients will likely not respond to UC treatment such as 5 Aminosalicylates (5 ASA) and/or corticosteroid treatment and their UC will get worse. Therefore, clinicians can decide to skip these standard treatments and proceed with more aggressive UC treatments. Disease progression means that UC can progress through the stages of disease. The main stages of UC are mild, moderate, severe, and fulminant.

The term "treatment" as used herein and associated terms such as "treat" and "treating" generally refers to obtaining a desired pharmacologic and/or physiologic effect and in particular means the reduction or inhibition of the progression, severity and/or duration of Ulcerative Colitis or at least one symptom thereof. The term 'treatment' therefore refers to any regimen that can benefit a subject. Treatment may include curative, alleviative, palliative or prophylactic effects. The effect may be prophylactic in terms of completely or partially preventing a disease or symptom thereof and/or may be therapeutic in terms of a partial or complete stabilization or cure for a disease and/or adverse effect attributable to the disease. "Treatment" as used herein covers any treatment of a disease in a mammal, particularly a human, and includes: (a) preventing the disease or symptom from occurring in a subject which may be predisposed to the disease or symptom but has not yet been diagnosed as having it; (b) inhibiting the disease symptom, i.e., arresting its development; or (c) relieving the disease symptom, i.e., causing regression of the disease or symptom. The term "therapeutic" does not necessarily imply that a subject is treated until total recovery. Similarly, "prophylactic" does not necessarily mean that the subject will not eventually contract a disease condition. Accordingly, therapeutic and/or prophylactic treatment includes amelioration of the symptoms of UC or preventing or otherwise reducing the risk of developing UC. The term "prophylactic" may be considered as reducing the severity or the onset of a particular condition. "Therapeutic" may also reduce the severity of an existing condition.

In the context of the present invention "prognosis", "prediction" or "predicting" should not be understood in an absolute sense, as in a certainty that an individual will develop Ulcerative Colitis (including UC progression), but as an increased risk or likelihood to develop UC or of UC progression. "Prognosis" is also used in the context of the invention for predicting UC progression, in particular to predict therapeutic results of a certain therapy of the UC. The prognosis of a therapy can e.g. be used to predict a chance of success or chance of reducing the severity of the disease to a certain level. The inventive marker sets may also be used to monitor a patient for the emergence of therapeutic results or positive disease progressions.

The term 'gene expression signature' as used herein is a single or combined group of genes with a uniquely characteristic pattern of gene expression that occurs as a result of an altered or unaltered biological process or pathogenic medical condition. The phenotypes that may theoretically be defined by a gene expression signature range from those that predict the survival or prognosis of an individual with a disease, those that are used to differentiate between different subtypes of a disease, to those that predict activation of a particular pathway. Ideally, gene signatures can be used to select a group of patients for whom a particular treatment will be effective. A "gene expression signature" is a dataset that has been obtained from RNA cells extracted from FFPE-derived patient tissue, and provides information on the change in expression of a set of genes. A useful signature may be obtained from all or a part of the gene dataset, usually the signature will comprise information from at least about 1 gene, at least about 2 genes, at least about 5 genes, at least about 8 genes, at least about 10 genes, at least about 15 genes, at least about 20 genes, at least about 25 genes, at least about 30 genes, at least about 34 genes or more, up to the complete dataset of the present invention. Where a subset of the dataset is used, the subset may comprise upregulated genes, downregulated genes, or a combination thereof. In particular, the gene expression signature of the present invention may comprise or consist of 34 upregulated genes.

The term "gene" refers to a nucleic acid (e.g., DNA) sequence that comprises coding sequences necessary for the production of a polypeptide, precursor, or RNA (e.g., rRNA, tRNA). The polypeptide can be encoded by a full length coding sequence or by any portion of the coding sequence so long as the desired activity or functional properties (e.g., enzymatic activity, ligand binding, signal transduction, immunogenicity, etc.) of the full-length or fragments are retained. The term also encompasses the coding region of a structural gene and the sequences located adjacent to the coding region on both the 5' and 3' ends for a distance of about 1 kb or more on either end such that the gene corresponds to the length of the full-length mRNA. Sequences located 5' of the coding region and present on the mRNA are referred to as 5' non-translated sequences. Sequences located 3' or downstream of the coding region and present on the mRNA are referred to as 3' non-translated sequences. The term "gene" encompasses both cDNA and genomic forms of a gene. A genomic form or clone of a gene contains the coding region interrupted with noncoding sequences termed "introns" or "intervening regions" or "intervening sequences." Introns are segments of a gene that are transcribed into nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide. A gene is declared 'differentially expressed' if a difference or change observed in read counts or expression levels/index between two experimental conditions is statistically significant. When the expression levels are significantly different, the corresponding genes are called Differentially Expressed Genes (DEGs). These DEGs are assumed to be the molecular driving force and/or the molecular biomarkers of different phenotypes.

The term "nucleic acid" is used broadly herein to mean a sequence of deoxyribonucleotides or ribonucleotides that are linked together by a phosphodiester bond. As such, the term "nucleic acid" or "nucleic acid sequence" is meant to include DNA and RNA, which can be single stranded or double stranded, as well as DNA/RNA hybrids. Furthermore, the term "nucleic acid" as used herein includes naturally occurring nucleic acid molecules, which can be isolated from a cell, as well as synthetic molecules, which can be prepared, for example, by methods of chemical synthesis or by enzymatic methods such as by the polymerase chain reaction (PCR), and, in various embodiments, can contain nucleotide analogs or a backbone bond other than a phosphodiester bond.

Any nucleic acid may be used in the present invention, given the presence of differently methylated CpG islands can be detected therein. The "CpG island" is a CpG-rich region in a nucleic acid sequence. A CpG island is capable of being differentially methylated. CpG islands have an average G*C content of about 60%, compared with the 40% average in bulk DNA. The islands take the form of stretches of DNA typically about one to two kilobases long. There are about 45,000 islands in the human genome. In many genes, the CpG islands begin just upstream of a promoter and extend downstream into the transcribed region. Methylation of a CpG island at a promoter usually suppresses expression of the gene. The islands can also surround the 5' region of the coding region of the gene as well as the 3' region of the coding region. Thus, CpG islands can be found in multiple regions of a nucleic acid sequence including upstream of coding sequences in a regulatory region including a promoter region, in the coding regions (e.g., exons), downstream of coding regions in, for example, enhancer regions, and in introns. Nucleic acids contained in a sample used for detection of methylated CpG islands may be extracted by a variety of techniques known to the person skilled in the art.

The terms "polynucleotide" and "oligonucleotide" also are used herein to refer to nucleic acid molecules. Although no specific distinction from each other or from "nucleic acid" is intended by the use of these terms, the term "polynucleotide" is used generally in reference to a nucleic acid molecule that encodes a polypeptide, or a peptide portion thereof, whereas the term "oligonucleotide" is used generally in reference to a nucleotide sequence useful as a probe, a PCR primer, an antisense molecule, or the like. Of course, it will be recognised that an "oligonucleotide" also can encode a peptide.

By `functionally active' is meant a nucleic acid sequence of a gene of the present invention that is capable of diagnosing Ulcerative Colitis or predicting the likelihood of disease progression requiring treatment beyond the standard treatments of 5 Aminosalicylates (5 ASA) and/or corticosteroids. Further, functionally active may indicate.a nucleic acid sequence of the genes of the present invention wherein the administration of a gene to a subject or expression of a gene in a subject promotes suppression of Ulcerative Colitis.

A 'fragment' can comprise at least 20, at least 50, at least 100 or at least 150 or greater contiguous amino acids from SEQ ID NOs:1-34 and which is functionally active. Suitably, a fragment may be determined using, for example, C-terminal serial deletion of cDNA. Said deletion constructs may then be cloned into suitable plasmids. The activity of these deletion mutants may then be tested for biological activity as described herein. Fragments may be generated using suitable molecular biology methods as known in the art.

By 'variant' is meant an amino acid sequence which is at least 90% homologous to SEQ ID NOs:1-34, even more preferably at least 95% homologous to SEQ ID NOs:1-34, even more preferably at least 96% homologous to SEQ ID NOs:1-34, even more preferably at least 97% homologous to SEQ ID NOs:1-34, and most preferably at least 98% homology with SEQ ID NOs:1-34. A variant encompasses a polypeptide sequence of SEQ ID NOs:1-34 which includes substitution of amino acids, especially a substitution(s) which is/are known for having a high probability of not leading to any significant modification of the biological activity or configuration, or folding, of the protein. These substitutions, typically known as conserved substitutions, are known in the art. For example the group of arginine, lysine and histidine are known interchangeable basic amino acids. Suitably, in embodiments amino acids of the same charge, size or hydrophobicity may be substituted with each other. Suitably, any substitution may be selected based on analysis of amino acid sequence alignments of interferon alpha subtypes to provide amino acid substitutions to amino acids which are present in other alpha subtypes at similar or identical positions when the sequences are aligned. Variants may be generated using suitable molecular biology methods as known in the art.

The term "diagnosed," as used herein, refers to the recognition of a disease by its signs and symptoms, or genetic analysis, pathological analysis, histological analysis, and the like. "Diagnosing" may or may not be used to provide information of a 100% sure Ulcerative Colitis presence, but is usually used to describe a risk or likelihood of Ulcerative Colitis presence. Such a risk or likelihood may be at least 60%, at least 70%, at least 80% at least 90% at least 95% or at least 98% probability.

The term "patient stratification" means methods of grouping or "stratifying" patients, according to the gene expression signature of the present invention. The present invention allows for the stratification of patients into groups such as progressors of UC disease and non-progressors of UC disease. To this purpose, the inventors defined disease progression as UC patients who required treatment escalation beyond 5 Aminosalicyclates (5 ASA) and/or corticosteroids. The progressors group comprise or consist of patients who test positive for the gene expression signature or a subset thereof and are likely to require treatment for UC beyond the standard 5-ASA treatment. In these cases, 5-ASA and/or corticosteroid treatment can be omitted and further UC treatments can be started at an early stage of disease progression.

The term 'antagonist' means a chemical substance that binds to and blocks the activation of certain receptors on cells, preventing a biological response. As used herein, the term 'antagonist' means any compound capable of blocking, attenuating, reducing, delaying or otherwise interfering with TFPI2 signalling and/or the interaction between TFPI2 and a cell surface receptor. A TFPI2 antagonist is also known as a TFPI2 inhibitor or TFPI2 blocker. Antagonists mediate their effects by binding to the active site or to the allosteric site on a receptor, or they may interact at unique binding sites not normally involved in the biological regulation of the receptor's activity. Antagonist activity may be reversible or irreversible depending on the longevity of the antagonist-receptor complex, which, in turn, depends on the nature of antagonist-receptor binding. The majority of drug antagonists achieve their potency by competing with endogenous ligands or substrates at structurally defined binding sites on receptors. Antagonists turn "off' a cellular response by 'blocking' the receptor from the agonist. A TFPI2 antagonist can be a small molecule, a protein, such as an antibody or fragment thereof, or a soluble TFPI2 receptor based trap, or a nucleic acid, such as an antisense molecule or an siRNA. A TFPI2 antagonist can be nucleic acids that inhibit the activity or expression (e.g., transcription, translation, secretion from a cell, or combinations thereof) of TFPI2 as well as small molecules that inhibit the activity or expression (e.g., transcription, translation, secretion from a cell, or combinations thereof) of TFPI2. As used herein, "an antibody that binds TFPI2" or an "anti- TFPI2 antibody" includes antibodies, and antigen-binding fragments thereof, that bind a human TFPI2 protein or a biologically active fragment thereof.

The term "antibody", as used herein, means any antigen-binding molecule or molecular complex comprising at least one complementarity determining region (CDR) that specifically binds to or interacts with a particular antigen (e.g., TFPI2). The term "antibody" includes immunoglobulin molecules comprising four polypeptide chains, two heavy (H) chains and two light (L) chains inter-connected by disulfide bonds, as well as multimers thereof (e.g., IgM). Each heavy chain comprises a heavy chain variable region (abbreviated herein as HCVR or V_{H}) and a heavy chain constant region. The heavy chain constant region comprises three domains, C_{H}1, C_{H}2 and C_{H}3.

Each light chain comprises a light chain variable region (abbreviated herein as LCVR or V_{L}) and a light chain constant region. The light chain constant region comprises one domain (Ci_1). The V_{H} and V_{L} regions can be further subdivided into regions of hypervariability, termed complementarity determining regions (CDRs), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. In different embodiments of the invention, the FRs of the anti-TFPI2antibody (or antigen-binding portion thereof) may be identical to the human germline sequences, or may be naturally or artificially modified. An amino acid consensus sequence may be defined based on a side-by-side analysis of two or more CDRs.

The term "antibody", as used herein, also includes antigen-binding fragments of full antibody molecules. The terms "antigen-binding portion" of an antibody, "antigen-binding fragment" of an antibody, and the like, as used herein, include any naturally occurring, enzymatically obtainable, synthetic, or genetically engineered polypeptide or glycoprotein that specifically binds an antigen to form a complex. Antigen-binding fragments of an antibody may be derived, e.g., from full antibody molecules using any suitable standard techniques such as proteolytic digestion or recombinant genetic engineering techniques involving the manipulation and expression of DNA encoding antibody variable and optionally constant domains. Such DNA is known and/or is readily available from, e.g., commercial sources, DNA libraries (including, e.g., phageantibody libraries), or can be synthesized. The DNA may be sequenced and manipulated chemically or by using molecular biology techniques, for example, to arrange one or more variable and/or constant domains into a suitable configuration, or to introduce codons, create cysteine residues, modify, add or delete amino acids, etc. Non-limiting examples of antigen-binding fragments include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (e.g., an isolated complementarity determining region (CDR) such as a CDR3 peptide), or a constrained FR3-CDR3-FR4 peptide. Other engineered molecules, such as domainspecific antibodies, single domain antibodies, domain-deleted antibodies, chimeric antibodies, CDR-grafted antibodies, diabodies, triabodies, tetrabodies, minibodies, nanobodies (e.g. monovalent nanobodies, bivalent nanobodies, etc.), small modular immunopharmaceuticals (SMIPs), and shark variable IgNAR domains, are also encompassed within the expression "antigen-binding fragment," as used herein.

'Pharmaceutical compositions' according to the present invention, and for use in accordance with the present invention, may comprise, in addition to an active ingredient, a pharmaceutically acceptable excipient, carrier, buffer stabiliser or other materials well known to those skilled in the art. Such materials should be non-toxic and should not interfere with the efficacy of the active ingredient. The precise nature of the carrier or other material will depend on the route of administration, which may be, for example, oral, intravenous, intranasal or via oral or nasal inhalation. The formulation may be a liquid, for example, a physiologic salt solution containing nonphosphate buffer at pH 6.8-7.6, or a lyophilised or freeze-dried powder.

The composition of the invention is typically administered to a subject in a "therapeutically effective amount", this being an amount sufficient to show benefit to the subject to whom the composition is administered. The actual dose administered, and rate and time-course of administration, will depend on, and can be determined with due reference to, the nature and severity of the condition which is being treated, as well as factors such as the age, sex and weight of the subject being treated, as well as the route of administration. Further due consideration should be given to the properties of the composition, for example, its binding activity and in-vivo plasma life, the concentration of the antibody or binding member in the formulation, as well as the route, site and rate of delivery.

Dosage regimens can include a single administration of the composition, or multiple administrative doses of the composition. The compositions can further be administered sequentially or separately with other therapeutics and medicaments which are used for the treatment of the condition for which the composition of the present invention is being administered to treat.

Examples of dosage regimens which can be administered to a subject can be selected from the group comprising, but not limited to; 1µg/kg/day through to 20mg/kg/day, 1µg/kg/day through to 10mg/kg/day, 10µg/kg/day through to 1mg/kg/day. In certain embodiments, the dosage will be such that a plasma concentration of from 1µg/ml to 100µg/ml of the DMR is obtained. However, the actual dose of the composition administered, and rate and time-course of administration, will depend on the nature and severity of the condition being treated. Prescription of treatment, e.g. decisions on dosage etc, is ultimately within the responsibility and at the discretion of general practitioners and other medical doctors, and typically takes account of the disorder to be treated, the condition of the individual patient, the site of delivery, the method of administration and other factors known to practitioners.

As used herein, the term "subject" refers to any organisms that are screened using the diagnostic methods described herein. Such organisms preferably include, but are not limited to, mammals (e.g., murines, simians, equines, bovines, porcines, canines, felines, and the like), and most preferably includes humans. The subject may have had Ulcerative Colitis or may have had no history of Ulcerative Colitis, in particular if the subject is suspected of having Ulcerative Colitis. The invention may be used as a routine test even without any suspect of the subject having Ulcerative Colitis. If the subject has UC, the invention may be used to determine the likelihood of the subject progressing to UC that requires treatment beyond the standard treatments of 5 ASA and/or corticosteroids.

A "biological sample" means a sample which comprises genomic DNA of the subject to be investigated, such as, for example, a blood sample, saliva sample, a smear, any other cell containing sample, etc. As used herein, the term "sample" is used in its broadest sense. In one sense, it is meant to include a specimen or culture obtained from any source, as well as biological and environmental samples. Biological samples may be obtained from animals (including humans) and encompass fluids, solids, tissues, and gases. Biological samples include blood products, such as plasma, serum and the like. Such examples are not however to be construed as limiting the sample types applicable to the present invention.

In the present invention, "normal" cells refer to those that do not show any abnormal morphological or cytological changes.

As used herein, the terms "detect", "detecting" or "detection" may describe either the general act of discovering or discerning or the specific observation of a detectably labeled composition.

Throughout the specification, unless the context demands otherwise, the terms 'comprise' or 'include', or variations such as 'comprises' or 'comprising', 'includes' or 'including' will be understood to imply the inclusion of a stated integer or group of integers, but not the exclusion of any other integer or group of integers.

Unless defined otherwise, all technical and scientific terms used herein have the same meaning as commonly understood to one of ordinary skill in the art to which this invention belongs. Although any methods, devices and materials similar or equivalent to those described herein can be used in the practice or testing of the invention, the preferred methods, devices and materials are now described.

### Brief description of the Figures

The invention will be more clearly understood from the following description of some embodiments thereof, given by way of example only, with reference to the following figure in which:
Figure 1A and Figure 1B demonstrate differentially expressed genes between progressor vs non-progressor UC patients. Differentially expressed genes identified following mRNAseq analysis for UC progressors (n=25) and non-pregressors (n=9) patients. Fig 1A is a volcano plot which demonstrates significantly (adjusted p<0.05) 34 up-regulated (logFold >0.584) in UC progressor patients relative to non-progressor UC patients. Figure 1B is a heatmap generated using 34 up-regulated (logFold >1.5) genes including unsupervised hierarchical clustering of progressor and non-progressor UC patients.
Figure 2 demonstrates cell type deconvolution comparison between progressor vs non-progressor UC patients. Immune/general type deconvolution carried out by applying mRNAseq data to MCP counter analysis showed significant increase in endothelial cells (adjusted p=0.002) and fibroblasts [adjusted p=0.004) in UC progressor patients (labelled: "pro") relative to non-progressor (labelled: "Npro") UC patients. The samples labelled "norm" in the graph were matched macrodissected histologically determined uninflamed tissue.
Figure 3A-C demonstrate identifying master regulator mediated signalling networks regulating up-regulated genes in UC progressor patients. Figure 3A shows that master regulators typically sit at the top of the hierarchy in a signalling network and regulate genes through intermediate signalling molecules followed by transcription factors. Identification of master regulators mediated signalling networks was carried out by applying genes up-regulated in UC progressor to the GeneXplain platform. Figure 3B shows that 3 master regulators (MRs) were identified to regulate expression of upregulated genes in concordant manner through 11 transcription factors. Figure 3C identifies these 11 transcription factors.

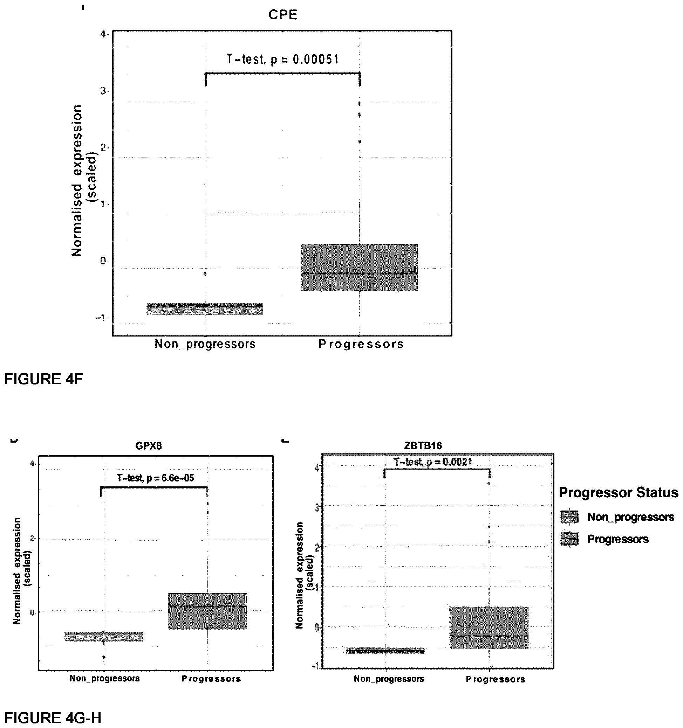

Figure 4 shows specific predictive power of three specific genes: *CPE, GPX8* and *ZBTB16.* Figure 4A shows the Lasso spectrum for the 34 upregulated DEGs shown on the y-axis against the log (lambda) sequence generated by a single-fold LASSO- penalized model shown on the x-axis. The colored curves denote the 34 DEGs upregulated in progresses. Figure 4B illustrates Optimal penalization coefficients lambda in the LASSO model as identified using ten-fold crossvalidation and the minimum criterion. The left vertical dotted line represents the minimum error, and the right line represents the cross-validated error within one standard error of the minimum. The upper Abscissa indicates the number of independent variables that still exist in the model. Figure 4C-E displays the ROC (Receiver Operator Curve) for the top predictors of disease progression in patients with UC. Finally, Figure F-H, shows boxplots of the normalised gene expression (scaled) of three genes (displaying most significant predictive power after LASSO analysis) showing statistically significant different between progressors and non-progressors adult UC patients.
Figure 5 demonstrates qPCT-based validation carried out for the top upregulated genes on RNA derived from treatment naive UC samples as well as samples derived from an independent validation cohort with identical characteristics as the discovery cohort. The predictive power if these genes is evaluated using AUC and ROC analysis.
Figure 6 demonstrates an analytical study workflow describing identifying master transcription regulators (MTRs) and transcription factors (TFs) and how to ascertain the phenotypic role of the MTRs in driving UC pathogenesis.
Figure 7 demonstrates upset plots of common transcription binding sites (TFBS) enriched within DEGs of four UC patient non-responders vs responders datasets. Intersection of upregulated TFBS showing 7 TFBS overlapping in over two datasets, RELA, NANOG and FOSJUN (circled) were the top binding sites. GSE12251 (orange), GSE73661 (purple), GSE23597 (sky-blue), GSE16879 (lilac).
Figure 8 demonstrates RT-qPCR of upregulated and downregulated MTRs in non-responders to IFX using CRL1831 stimulated cells. Figure 8A shows TFPI2 upregulated MTRs significant between unstimulated and stimulated (p=0.03). Figure 8B shows CXCL8 upregulated MTRs not significant between unstimulated and stimulated (p=0.051). Figure 8C shows SELE upregulated MTRs not significant between unstimulated and stimulated (p=0.99). Figure 8D shows FCGR3B upregulated MTRs not significant between unstimulated and stimulated (p=0.55). Figure 8E shows FGFR2 downregulated MTRs significant between unstimulated and stimulated (p=0.01). Housekeeper gene used endogenous 18s. Unstimulated (purple), stimulated (orange).
Figure 9 demonstrates knockdown of significant TFPI2 MTR upregulated in non-responders using CRL1831 stimulated cells. *TFPI2* knockdown with concentration at 25 nM and measured at 72 hr post-TNFA stimulation was significant in comparison to scrambled control (non-target siRNA). *n*= *3, error bars represent SEM. *** p*< *0.001.* All expression data normalised to 18s as endogenous control.
Figure 10 demonstrates *TFPI2* knockdown had significant on the expression of in-silico identified target genes in CRL1831. Cells were transfected with 25 nM siRNA using TFPI2 and incubated for 72 hours before examining the mRNA expression of the target genes. Scrambled control (non-target siRNA). **p*< *0.05; **p*<*0.01; ***p*< *0.001, student's t-test, unpaired.* Housekeeper gene used endogenous 18s. *A)C5AR1, B)TNFAPI3, C)TNFAPI6, D)FCGR3B, E)BIRC3, F)CLCX5.*
Figure 11 demonstrates cytokine expression level (pg/ml) after TFPI2 knockdown CRL1831 stimulated cells. Concentrations of IFNy (Figure 11A), IL10 (Figure 11B), IL13 (Figure 11C), IL1β (Figure 11D), IL2 (Figure 11E), IL6 (Figure 11F), TNFα (Figure 11G), and IL8 (Figure 11H) were measured in CRL1831 media after 48h from TFPI2 silencing. The average (error bars ± SEM) of three independent triplicate experiments is shown (n3). Concentrations were plotted in pg/ml.
Figure 12 demonstrates reactome analysis of DEGs represented by the dot plot. Figure 12A demonstrates upregulated pathways and Figure 12B demonstrates downregulated pathways.
Figure 13 demonstrates KEGG analysis of DEGs represented by the dot plot. Figure 13A demonstrates upregulated pathways and Figure 13B demonstrates downregulated pathways.

### Experimental Data

### Material and methods

### 1. RNA and DNA Extraction and Quantification

RNA was extracted from FFPE-derived patient tissue. To deparaffinise the FFPE tissue embedded slides, slides were baked at 60°C for 1 hour. After baking, the slides were then washed in Xylene, followed by serial dehydration in 100% ethanol (twice), 90% ethanol, 80% ethanol, 70% ethanol and rehydration in water (twice). Each wash was 2 min long. Once the washes were complete, samples were scraped off the slides using a scalpel, and placed in labelled Eppendorf tubes. Both DNA and RNA was extracted from the samples using the Norgen extraction kits (Norgen, ON, Canada). After extraction, Nanodrop 2000 was used to quantify the RNA and DNA.

### cDNA Synthesis

A master mix containing 10X reaction buffer, 25X dNTP Mix (mM), 10X reaction random primers, MultiScribe^{™} Reverse Transcriptase, RNase Inhibitor and nuclease-free water was made. 10µl of the master mix was added to the RNA samples, totalling to 20µl per sample reaction mix. cDNA was synthesised using the highcapacity cDNA reverse transcription kit (Invitrogen) as per the manufacturer's instructions. Samples were incubated in the Bio-Trad T100 Thermal Cycler (BioRad) at 25°C for 10min, followed by 37°C for 60min and 85°C for 5min.

### Quantitative polymerase chain reaction (qPCR)

A primer master mix was prepared for each primer set to yield 10µM of each forward and reverse primer. A master mix containing 6.6µl of nuclease-free water, 1.2µl forward primer, 1.2µl reverse primer and 10µl SYBR green (Promega) was made. To each cDNA (1µl) sample, 19µl of the master mix was added in triplicate to the appropriate number of wells in a 96-well plate. The plate was sealed, centrifuged briefly in a benchtop centrifuge to spin down the contents of the well, and placed into an Applied Biosystems 7500 real time PCR system set to the following temperature cycles: 2 min at 50°C, 10 min at 95°C, followed by 40 cycles oscillating between 15 seconds at 95°C and 1 min at 60°C. A melt curve was generated after each run in order to ensure primer specificity: 15 seconds at 95°C, 15 seconds at 60°C and 15 seconds at 95°C.

### RNA Sequencing

First, RNA quality was ascertained using the AATI fragment analyser. Next, RNA libraries were generated using the Kapa RNA hyperprep library preparation kit (Kapa Biosystems) and subsequently quantified using qPCR and pooled in equimolar amounts. The pooled libraries were sequenced on the Illlumina NovaSeq 600 platform (2 x 50bp) at the Genomics Core Facility, Queen's University Belfast, U.K.

### 2. RNA Analysis

### Differentially expressed genes analysis

The differential expression of genes in this study was analysed using DESeq2. DESeq2 uses non-linear model and was utilised to perform normalisation and DEG analysis. The input for this analysis was provided by the nf-core/rna-seq pipeline through the use of DESeqDataSet. The results were then visualized through the use of volcano plots in ggplot2 R package, providing a clear representation of the gene's fold changes and associated p-values. Prior to implementing DESeq2, our dataset underwent pre-processing procedures. Using the Biomart package in R, each gene was meticulously annotated and non-protein coding genes were eliminated. Strict filtering criteria was applied to all protein coding genes by removing those with low counts, as they often result in false positives. This involved calculating the median of each gene row and removing those with values equal to zero. Additionally, sequence batch effects and site collections effects (Beaumont Hospital and St. Vincent Hospital) were adjusted for using the 'sva' function in the Combat package in R.

Differentially expressed genes were identified by using a combination of criteria, including fold change ± 1.5 and adjusted p-value ≤ 0.05.

### Enrichment analysis

Genes displaying differential expression were subjected to gene ontology (GO) enrichment analysis in order to detect alterations in cellular pathways. This was carried out by utilising the Database for Annotation, Visualization and Integrated Discovery (DAVID) tool, for GO, Kyoto Encyclopedia of Genes and Genomes (KEGG) and REACTOME pathways. The DEGs that were classified as either upregulated or downregulated were inputted into DAVID for enrichment analysis. A threshold of ≤ 0.05 FOR was considered statistically significant. The dotplots for GO, KEGG, and Reactome pathways were generated using the ggplot2 software.

### Machine learning for predictive modelling

A 'least absolute shrinkage and selection operator' (LASSO) logistic regression model to determine the most effective gene expression biomarkers for predicting diagnosis. This approach utilized the penalized maximum likelihood function (*'glmnet'*) and crossvalidation glmnet function (*'cv.glmnet'*) from the glmnet package within the generalized linear model framework. The ROC curves were produced utilising the R package pROC, with the purpose of evaluating diagnostic accuracy. The area under the curve (AUC) was then computed to assess this accuracy. An AUC value exceeding 0.8 is indicative of a well-fitted model.

### 3. Identification of Transcription factors and master transcriptional regulators

### Genome Enhancer Workflow

Using the Genome Enhancer platform provided by GeneXplain, a promoter and upstream network analysis of DEGs to uncover potential transcription factors (TFs) and master transcriptional regulators MTRs was conducted.The initial stage in Genome Enhancer analysis involved generating a collection of genes that accurately represent the pathological process under study. Next, the regulatory regions (such as promoters and enhancers) associated with our genes of interest were identified and mapped out. The regulatory regions were then scanned for enrichment of TF binding sites. Subsequently, using the TRANSFAC^{®} database and Match and Composite Module Analyst (CMA) algorithms, modules of co-acting TFs are identified. Next, the Genome Enhancer pipeline performed network analysis in order to identify the crucial MTRs of the specific pathological process being targeted.

### I. To utilise Genome Enhancer, we followed the following steps:

- Uploaded our data to the server and selected the import options according to the data type.
- Separated our data based on the desired comparison conditions.
- Initiated the analysis by specifying the comparison conditions, as well as any optional disease and tissue types.

### II. Identification of genes controlled by master regulators based on the input gene list

To determine which genes are regulated by the identified MTRs, a downstream analysis was conducted. We first identified the corresponding TFs for each MTR and then determined the associated genes. The "sites" column in the "model visualisation on the yes set" table was filtered based on the top three TFBS/TFs from each analysis. The resulting genes (Names column) were saved and compared for any overlapping commonalities.

### Microenvironment Cell Populations-counter (MCP-counter) analysis

The "MCPcounter" package in R was used for analysis of microenvironment cell populations and quantification of immune cells from transcriptomic data. This method estimates the abundance of different immune cells based on specific molecular markers. The visualization of immune cell infiltration of boxplot and heatmap was facilitated by use of the "ggplot2" package in R.

### 4. Prediction of IFX non-responders in UC

The inventors used a microarray meta-analysis approach to predict IFX non-responders from responders in UC. To that purpose, the inventors conducted a thorough search for microarray datasets which investigated differentially expressed genes between responders and non-responders to anti-TNF therapy in patients with inflammatory bowel disease. The search was performed on the NCBI Gene Expression Omnibus (GEO) using keywords such as "anti-TNF", "Infliximab", "inflammatory bowel disease", "IBD", "ulcerative colitis", "UC", "before therapy", "after therapy". The selection criteria for GEO datasets included in this analysis are as follows: (1) The samples must be from human subjects. (2) Raw data of both responders and non-responders at baseline. (3) All included datasets must be publicly accessible. From the eligible studies, the following information was extracted: (1) GEO accession number, (2) platform used, (3) sample type, (4) UC only, and (5) number of non-responders and responders. The gene expression datasets were downloaded and pre-processed and analysed in R using LIMMA package to identify differentially expressed genes (Figure 1). Significant genes were identified using cut-off from literature of LogFC ± 1.5 and adjusted *p*-value ≤ 0.05. Pathways associated with significant genes were identified using DAVID (Database for Annotation, Visualization and Integrated Discovery). Next, GeneXplain platform was used in the identification of MTRs and TFs (as described in the previous section).

### Development of IBD-like in vitro model

CRL-1831 (normal colon epithelial) (ATCC^{®} CRL-1831TM) cell line purchased from American Type Culture Collection (ATCC) (Manassas, VA 20108 USA) was established from a normal fetal colonic mucosa (13 weeks gestation). CRL-1831 cells were cultured in DMEM/F12 medium supplemented with 10 ng/ml cholera toxin, 0.005 mg/ml insulin (Sigma Aldrich), 0.005 mg/ml transferrin, 100 ng/ml hydrocortisone, 20 ng/ml human recombinant EGF, and 10% Fetal Bovine Serum (Gibco, Invitrogen). Cells were maintained at 37 °C in an incubator at 5% CO₂ and grown to -70% confluency. They were tested for mycoplasma contamination using a MycoAlert detection kit (Lonza, UK). The cells were then plated in a 6-well plate and stimulated with 20 ng of TNFα for 24 hrs. This resulted in significant upregulation of pro-inflammatory cytokines such as IL8 and IL2p70 (as measured by ELISA).

### Enzyme-linked immunosorbent assay (ELISA)

The supernatant stored from TNF stimulated and treated cells were used for measuring Interleukins IL-8, IL-6, TNF and IL-12/IL-23p40 production using Enzyme-linked immunosorbent assay (ELISA) ((DuoSet, R&D systems, a biotech brand, Europe), DY406 (IL-6), DY410 (TNF), DY208 (IL-8), DY1240 (IL-12/IL-23p40)) with absorbance read using a microplate reader at 450 nm and compared against a standard curve. Samples were diluted according to kit protocols and assessed in triplicate. Alternatively, the Mesoscale MultiSPOT platform (MesoScale Diagnostics LLC., Rockville, MD, USA) assay was used to examine a panel of cytokines (TNFα, IFNγ, IL-1β, IL-2, IL-4, IL-6, IL-8, IL-10, IL-12p70, IL-13), according to manufacturers' instructions. All samples were assessed in duplicate and replicates with coefficient of variance > 20% or out of range were excluded from downstream analysis.

### siRNA transfection

For transfection, cells were seeded and stimulated (TNF) in a 6-well plate for 24 H. the ON-TARGETplus siRNAs (20 TFPI2 (L-017683-00-0005), GAPD (D-001830-10-05), Non-targeting (D-001810-10-05)) (Dharmacon reagents, Horizon Discovery Ltd., Ireland) were individually diluted in serum-free DMEM (SFM) up to a volume of 200 µl prior to combination with DharmaFECT transfection reagent (T-2001-03). DharmaFECT reagent was diluted in SFM to a total of 200 µl for each siRNA, and both mixtures were left to incubate at room temperature for 5 min. Next, each siRNA mixture was combined with DharmaFECT reagent and incubated for 20 min at room temperature to allow transfection/siRNA complexes to form. During this time, media was removed from each well and replaced with 1600 µl of fresh SFM. Hence, 400 µl of each transfection-siRNA complex was gently added dropwise to each well and the plate was rocked back and forth to mix. Cells were incubated at 37 °C for another 24 H. After 24 H, transfected media was removed, replaced with fresh growth media and incubated for 72 H. Knockdown of TFPI2 was determined by qPCR and Mesoscale MultiSPOT platform ELISA (as described above).

siRNA-mediated knock-down select MTRs in normal rectal epithelial cell line - CRL1831 stimulated with TNFα (IBD-like *in vitro* model) following by multiplex cytokine ELISA as well as qRT-PCR to ascertain the phenotypic role of the MTRs in driving UC pathogenesis.

Various modifications and variations to the described embodiments of the inventions will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes of carrying out the invention which are obvious to those skilled in the art are intended to be covered by the present invention.

### References

1. Conrad K, Roggenbuck D, Laass MW. Diagnosis and classification of ulcerative colitis. Autoimmun Rev 2014; 13: 463-6.
2. Ungaro R, Mehandru S, Allen PB, Peyrin-Biroulet L, Colombel JF. Ulcerative colitis. Lancet 2017; 389: 1756-70
3. da Silva BC, Lyra AC, Rocha R, Santana GO. Epidemiology, demographic characteristics and prognostic predictors of ulcerative colitis. World J Gastroenterol 2014; 20: 9458-67
4. Pillai N, Dusheiko M, Burnand B, Pittet V. A systematic review of cost-effectiveness studies comparing conventional, biological and surgical interventions for inflammatory bowel disease. PLoS One 2017; 12: e0185500
5. Pillai N, Dusheiko M, Burnand B, Pittet V. A systematic review of cost-effectiveness studies comparing conventional, biological and surgical interventions for inflammatory bowel disease. PLoS One 2017; 12: e0185500
6. Stevens TRJ, James JP, Simmonds NJ, McCarthy DA, Laurenson IF, Maddison PJ, Rampton DS. Circulating von Willebrand factor in inflammatory bowel disease. Gut 1992; 33: 502-506
7. Abozied A, Ahmed YA, Mohammed Saleh MF, Galal H, Abbas WA. Assessment of Von Willebrand factor antigen and activity levels in inflammatory bowel diseases. Egyptian journal of haematology 2021; 46(4): 227-233.
8. Arijs I, Li K, Toedter G, Quintens R, Van Lommel L, Van Steen K, Leemans P, De Hertogh G, Lemaire K, Ferrante M, Schnitzler F, Thorrez L, Ma K, Song XY, Marano C, Van Assche G, Vermeire S, Geboes K, Schuit F, Baribaud F, Rutgeerts P. Mucosal gene signatures to predict response to infliximab in patients with ulcerative colitis. Gut 2009; 58: 1612-9.
9. Bermejo F, Algaba A, Bonillo D, Jimenez L, Guardiola-Arevalo A, Pacheco M, Castano A, Guerra I. Limitations of the determination of faecal calprotectin in patients with ulcerative colitis and inflammatory polyps. Gastroenterol Hepatol 2020; 43: 73-8.
10. Chen F, Hu Y, Fan YH, Lv B. Clinical Value of Fecal Calprotectin in Predicting Mucosal Healing in Patients With Ulcerative Colitis. Front Med (Lausanne) 2021; 8: 679264.

## Claims

1. A method for predicting the progression of Ulcerative Colitis in a subject and/or screening for an increased likelihood of progression of Ulcerative Colitis in a subject, the method comprising:
- providing a biological sample which has been obtained from the subject;
- testing the biological sample for the presence or absence of a gene expression signature or a subset thereof;
wherein the gene expression signature, or a subset thereof, comprises or consists of one or more of the genes set forth in Table 1;
wherein the presence of the gene expression signature, or a subset thereof, indicates an increased likelihood of disease progression.

2. A method of using a gene expression signature or a subset thereof in a subject as a biomarker to evaluate the likelihood that a therapy for Ulcerative Colitis would be effective in the subject, the method comprising:
- providing a biological sample which has been obtained from the subject; and
- testing the biological sample for the presence or absence of a gene expression signature or a subset thereof;
wherein the gene expression signature or a subset thereof comprises or consists of one or more of the genes listed in Table 1;
wherein the presence of the gene expression signature or a subset thereof indicates that the therapy should be avoided.

3. A method of predicting the responsiveness of a patient suffering from UC to a particular treatment or a method for determining if the treatment of a patient with UC should be escalated, the method comprising:
- providing a biological sample which has been obtained from the subject; and
- testing the biological sample for the presence or absence of a gene expression signature or a subset thereof;
wherein the gene expression signature or a subset thereof comprises or consists of one or more of the genes listed in Table 1;
wherein the presence of the gene expression signature or a subset thereof indicates that the therapy should be avoided.

4. The method of claims 2 or 3, wherein the therapy is 5-aminosalicylate acid (5-ASA) and/or corticosteroid treatment.

5. The method of claims 1 to 4, wherein the gene expression signature or a subset thereof comprises or consists of one or more genes selected from the group comprising or consisting of carboxypeptidase E (*CPE*), WAP four-disulfide core domain 1 (*WFDC1*), Calsequestrin 2 (*CASQ2*), LIM domain only 3 (*LMO3*), Synemin (*SYNM*), Actin Gamma 2, smooth muscle (*ACTG2*), Patched domain containing 1 (*PTCHD1*), Heat Shock protein family B (small) member 7 *(HSPB7),* Filamin C (*FLNC*)*,* Carboxypeptidase X, M14 family member 2 (*CPXM2*)*,* Methionine sulfoxide reductase B3 (*MSRB3*)*,* Actin alpha 2, smooth muscle (*ACTA2*), Monoamine oxidase B (*MAOB*)*,* Nexilin F-actin binding protein (*NEXN*), Destrin, actin depolymerizing factor (*DSTN*), Prostaglandin-endoperoxide synthase 1 *(PTGS1),* Kelch like family member 23 (*KLHL23*)*,* Rho GTPase activating protein 10 *(ARHGAP10),* Mitogen-activated protein kinase kinase kinase 20 (*MAP3K20*)*,* Glutathione peroxidase 8 (GPX8), Tensin 1 (*TNS1*)*,* Slit guidance ligand 2 (*SLIT2*)*,* Tropomyosin 2 *(TPM2),* Caldesmon 1 (*CALD1*), Receptor activity modifying protein 1 (*RAMP1*)*,* Transgelin (*TAGLN*), Myosin Light chain 9 (*MYL9*)*,* Microtubule associated protein 1A (*MAP1A*)*,* Zinc finger and BTB domain containing 16 (*ZBTB16*)*,* Secreted frizzled related protein 1 (*SFRP1*)*,* Cyclase associated actin cytoskeleton regulatory protein 2 (*CAP2*), Myosin light chain kinase (*MYLK*)*,* Myomesin 1 (*MYOM1*) and RAS oncogene family (*RAB23*)*.*

6. The method of claims 1 to 5, wherein the gene expression signature comprises or consists of the mRNA sequences of the one or more genes and the mRNA sequences comprise or consist of the nucleic acid sequences of SEQ ID NO:1 to SEQ ID NO:34 or functionally active fragments or variants thereof; or any combination thereof.

7. The method of claims 1 to 6, wherein the gene expression signature comprises or consists of one or more genes selected from the group comprising or consisting of Carboxypeptidase E (*CPE*), Glutathione Peroxidase 8 (*GPX8*) and Zinc Finger and BTB Domain Containing 16 (*ZBTB16*)*.*

8. The method of claim 7, wherein the gene expression signature comprises or consists of the mRNA sequences of the one or more genes and the mRNA sequences comprise or consist of the nucleic acid sequences of SEQ ID NO:1 or a functionally active fragment or variant thereof, SEQ ID NO:20 or a functionally active fragment or variant thereof or SEQ ID NO:29 or a functionally active fragment or variant thereof; or any combination of SEQ ID NO:1, SEQ ID NO:20 or SEQ ID NO:29 or a functionally active fragments or variants thereof.

9. The method of claims 1 to 6, wherein the gene expression signature comprises or consists of one or more genes selected from the group comprising or consisting of Mitogen-activated protein kinase kinase 20 (*MAP3K20*)*,* Rho GTPase activating protein 1 (*ARHGAP10*) and Prostaglandin-endoperoxide synthase 1 (*PTGS1*)*.*

10. The method of claim 9, wherein the gene expression signature comprises or consists of the mRNA sequences of the one or more genes and the mRNA sequences comprise or consist of the nucleic acid sequences of SEQ ID NO:19 or a functionally active fragment or variant thereof, SEQ ID NO:18 or a functionally active fragment or variant thereof or SEQ ID NO:16 or a functionally active fragment or variant thereof; or any combination of SEQ ID NO:19, SEQ ID NO:18 or SEQ ID NO:16 or a functionally active fragments or variants thereof.

11. The method of claims 1 to 10, wherein the progression of Ulcerative Colitis is defined as subjects who require treatment that extends beyond 5 Aminosalicylates (5-ASA) and/or corticosteroid treatment.

12. A method of treatment and/or prophylaxis of Ulcerative Colitis in a subject in need thereof, the method comprising the steps of:
(a) determining the presence or absence of a gene expression signature or a subset thereof in a biological sample obtained from said subject;
wherein the gene expression signature or a subset thereof comprises or consists of one or more of the genes listed in Table 1;
wherein the presence of the gene expression signature or a subset thereof indicates an increased likelihood of disease progression in the subject from which the test sample was obtained; and
(b) administering to said subject an effective amount of an Ulcerative Colitis therapeutic agent.

13. The method of claim 12, wherein the therapeutic agent is an anti-TNFα targeting agent, preferably wherein the anti-TNFα targeting agent is infliximab, adalimumab or golimumab.

14. A Tissue Factor Pathway Inhibitor 2 (TFPI2) antagonist for use in the treatment and/or prophylaxis of Ulcerative Colitis, wherein the use of TFPI2 results in an improved response of a subject to an Ulcerative Colitis therapeutic agent.

15. A composition for use in the treatment and/or prophylaxis of Ulcerative Colitis, said composition comprising (i) a Tissue Factor Pathway Inhibitor 2 (TFPI2) antagonist; and (ii) a therapeutically effective amount of an Ulcerative Colitis therapeutic agent.
